# EUROPÄISCHE PATENTANMELDUNG

(11) **EP 2 803 364 A1**
(43) Veröffentlichungstag der Anmeldung: **19.11.2014**
(21) Anmeldenummer: 14401061.8
(22) Anmeldetag: 16.05.2014
(51) Int. Cl.: A61K 39/395, A61K 35/76, A61P 35/00, A61K 35/74

(54) **Pharmazeutisches Kombinationspräparat mit einem anti-idiotypischen Antikörperfragment**

(30) Priorität: 17.05.2013 DE 102013105144
(71) Anmelder: Thaller, Arno, 91801 Markt Berolzheim (DE)
(72) Erfinder: Thaller, Arno, 91801 Markt Berolzheim (DE)
(74) Vertreter: Heyerhoff Geiger & Partner Patentanwälte

(57) **Zusammenfassung**

Die Erfindung geht aus von einem pharmazeutischen Kombinationspräparat (10, 10', 10'') zu einer Unterdrückung einer humoralen Immunantwort gegen einen therapeutischen Organismus (24), enthaltend:
- Zumindest ein anti-idiotypisches Antikörperfragment (12) mit zumindest einer Erkennungsstelle (14), welche spezifisch zumindest eine Struktur (16) eines variablen Teils (18) eines weiteren Antikörperfragments (20) erkennt, wobei der variable Teil (18) des weiteren Antikörperfragments (20) so ausgeführt ist, dass er zum Erkennen von zumindest einem Epitop (22) zumindest des Organismus (24) vorbereitet ist; und
- mindestens einen pharmazeutisch verträglichen Exzipienten zumindest für das zumindest eine anti-idiotypische Antikörperfragment (12) und/oder mindestens ein pharmazeutisch verträgliches Vehikel zumindest für das zumindest eine anti-idiotypische Antikörperfragment (12).

Die Erfindung geht ferner aus von einem anti-idiotypischen Antikörperfragment (12) zur Verwendung als Medikament und von einem Polynukleotid codierend das anti-idiotypische Antikörperfragment (12).

## Beschreibung

Die Erfindung betrifft ein pharmazeutisches Kombinationspräparat mit zumindest einem anti-idiotypischen Antikörperfragment sowie das entsprechende anti-idiotypische Antikörperfragment zur Verwendung als Medikament und ein Polynukleotid codierend ein solches anti-idiotypisches Antikörperfragment.

Jährlich sterben allein in Deutschland 200.000 Menschen an Krebs. Sie versterben zumeist an den Folgen einer Metastasierung. Seit drei Jahrzehnten hat sich trotz Forschungsbillionen in diesem Stadium kein Fortschritt in der Krebstherapie ereignet. Die Betroffenen sterben trotz massiven Einsatzes von Chemotherapeutika und neuerer, sogenannter zielgerichteter, Moleküle, die jedes Budget sprengen. Beispielsweise kosten drei Injektionen des monoklonalen Antikörpers Ipilimumab, der das Alterungsepitop A4 auf zytotoxischen Lymphozyten hemmen soll, 120.000.- Euro und verlängern das mediane Überleben lediglich um drei Monate. Hinzu kommt nachteilig, dass eine solche Therapie allseits bekannte und erhebliche Nebenwirkungen aufweist.

Die Jahreskosten dieser Chemotherapien im weitesten Sinne belaufen sich mittlerweile auf weit über 60 Mrd. Euro. Ihr Anteil am Fünfjahresüberleben ist auf 2,7% geschätzt worden. Dies ist ein großes Missverhältnis zwischen Aufwand und Nutzen und erscheint kaum vorstellbar, noch vertretbar zu sein.

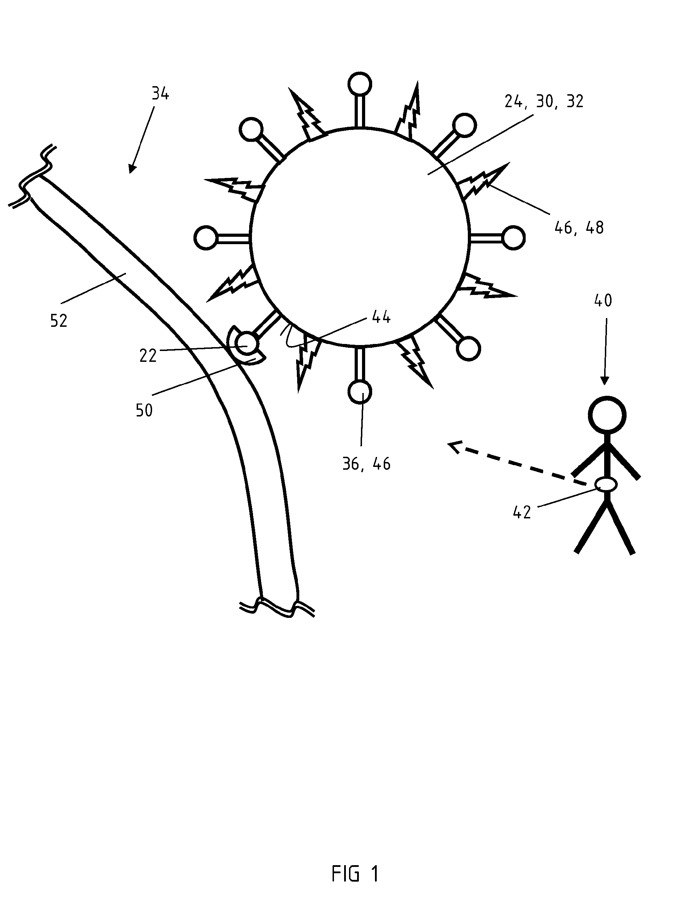

Es hat sich gezeigt, dass es sinnvoll ist, sich an komplexen Strategien zu orientieren, welche die Natur selbst gegen den sogenannten Krebs in Jahrmillionen hervorgebracht hat. Zu diesen natürlichen Krebsstrategien zählen zum einen das körpereigene Immunsystem und beispielsweise onkolytische Viren (vgl. FIG 1, genaue Beschreibung siehe Figurenbeschreibung). Leider wurde gefunden, dass diese beiden Strategien nicht in jeder Hinsicht "Hand in Hand" arbeiten. Das Immunsystem betrachtet auch die heilsamen Mikroorganismen als Feinde und mobilisiert dagegen das zelluläre wie das humorale Abwehrsystem durch Bildung von zytotoxischen T-Zellen und Antikörpern.

Die Bildung zytotoxischer T-Zellen gegen Virusepitope scheint unproblematisch zu sein. Sie greifen ja nicht die Viren selbst, sondern die Virus-infizierte Tumorzelle an. Der zelluläre Teil des Immunsystems wirkt also durchaus synergistisch mit onkolytischen Viren.

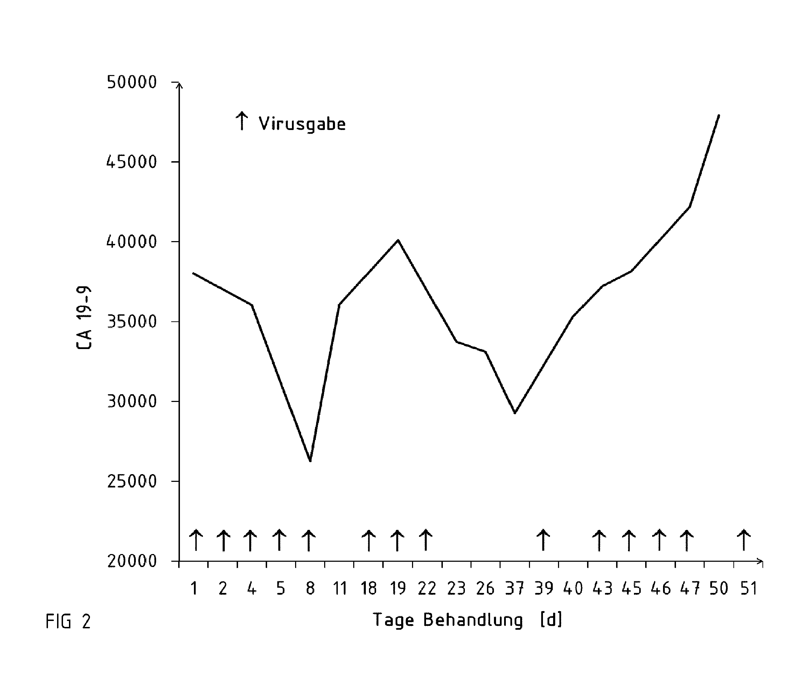

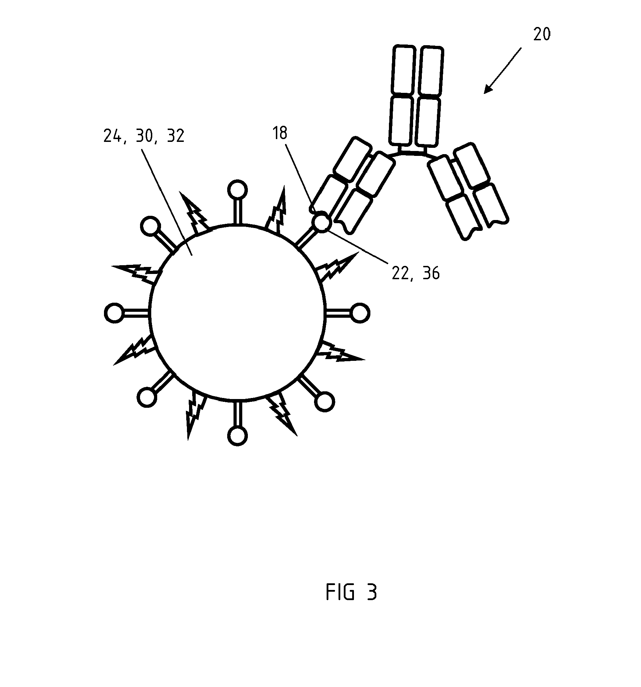

Die gebildeten Antikörper gegen onkolytische Viren hingegen fangen einen großen Teil der Viren ab, bevor diese ihre Zielstruktur, also eine Tumorzelle, erreichen können (vgl. FIG 3, genaue Beschreibung siehe Figurenbeschreibung). Darum kann bei einer Behandlung mit onkolytischen Viren, einer sogenannten Virotherapie, nicht selten beobachtet werden, dass der Patient auf die Virotherapie, überwacht durch einen Tumormarkerverlauf, anfangs gut anspricht, aber bereits nach wenigen Wochen deutlich weniger Erfolg oder gar kein Erfolg mehr erzielbar ist (vgl. FIG 2, genaue Beschreibung siehe Figurenbeschreibung).

Neben der Selektionierung resistenter Klone an Tumorzellen dürfte die Bildung von Antikörpern gegen Virusepitope die Hauptursache für dieses "Escape"-Phänomen sein. Diese Antikörper müssen nach Ende der Virusinfektion wieder herunter reguliert werden. Im Körper geschieht dies als physiologischer Vorgang, indem sogenannte anti-idiotypische Antikörper, also Anti-Antikörper, gebildet werden. Diese physiologische Gegenregulation ist aber so lange unwirksam, wie Viren zugeführt werden, was bei fortgeschrittener Krebserkrankung meist jahrelang nötig ist.

Um das Problem der Antikörper zu lösen, ist aus dem Stand der Technik bekannt, durch gleichzeitige Gabe von Zytostatika, wie bspw. Cyclophosphamid, die Antikörperbildung gegen Virusepitope zu unterdrücken. Dabei werden ca. 250 mg Wirkstoff benötigt. Diese Dosis ist zwar nur ein Bruchteil der zytostatischen Dosis, aber fünfmal so hoch wie bei einer üblichen Niedrigdosis-Chemotherapie, die zur Hemmung regulatorischer T-Zellen eingesetzt wird. Bei 250 mg täglich über lange Zeit sind deutliche Nebenwirkungen zu erwarten. Diese Art der Immuntherapie ist außerdem völlig unspezifisch: Zytostatika unterdrücken alle Antikörper und - bei dieser Dosis - nicht nur die regulatorischen, sondern auch die zytotoxischen T-Zellen und Natürlichen Killerzellen, die eine wichtige Rolle bei der Tumor-Abwehr spielen.

Es ist eine Aufgabe der vorliegenden Erfindung, ein pharmazeutisches Kombinationspräparat, ein anti-idiotypisches Antikörperfragment sowie ein dieses codierendes Polynukleotid bereitzustellen, bei deren Einsatz nachteilige Nebenwirkungen minimiert werden können und eine hohe Erfolgsquote in der Behandlung von Krebserkrankungen erzielt werden kann.

Diese Aufgabe wird erfindungsgemäß gelöst mit einem pharmazeutischen Kombinationspräparat mit den Merkmalen des Anspruchs 1 und einem anti-idiotypischen Antikörperfragment mit den Merkmalen des Anspruchs 14 sowie einem Polynukleotid mit den Merkmalen des Anspruchs 15. Günstige Ausgestaltungen und Vorteile der Erfindung ergeben sich aus den weiteren Ansprüchen und der Beschreibung.

Die Erfindung geht aus von einem pharmazeutischen Kombinationspräparat zu einer Unterdrückung einer humoralen Immunantwort gegen einen therapeutischen Organismus enthaltend: Zumindest ein anti-idiotypisches Antikörperfragment mit zumindest einer Erkennungsstelle, welche spezifisch zumindest eine Struktur eines variablen Teils eines weiteren Antikörperfragments erkennt, wobei der variable Teil des weiteren Antikörperfragments so ausgeführt ist, dass er zum Erkennen von zumindest einem Epitop zumindest des Organismus vorbereitet ist; und mindestens einen pharmazeutisch verträglichen Exzipienten zumindest für das zumindest eine anti-idiotypische Antikörperfragment und/oder mindestens ein pharmazeutisch verträgliches Vehikel zumindest für das zumindest eine anti-idiotypische Antikörperfragment.

Durch die erfindungsgemäße Ausgestaltung kann ein Kombinationspräparat zur Verfügung gestellt werden, welches eine hohe Spezifität für seine Bindungspartner hat. Dadurch ist es besonders gut verträglich und weist geringe bis keine Nebenwirkungen auf. In Folge dessen hat es eine besonders hohe Erfolgsquote in einer Behandlung von Erkrankungen, bei denen gegen den zur Behandlung eingesetzten Organismus Immunreaktionen des Empfängerorganismus ausgelöst werden, wie beispielsweise im Zusammenhang mit Krebserkrankungen. Damit kann das Kombinationspräparat beispielsweise erfolgreich die Nachfolge der Chemotherapie antreten, deren Möglichkeiten schon seit geraumer Zeit bis zur Grenze der "Menschlichkeit" ausgereizt sind.

Unter einem Kombinationspräparat soll ein Präparat und/oder Mittel verstanden werden, dass zumindest einen pharmazeutisch relevanten Bestandteil und/oder zumindest einen behandlungsrelevanten Bestandteil aufweist. Ein behandlungsrelevanter Bestandteil stellt insbesondere zumindest einen Stoff, ein Medikament und/oder ein Mittel dar, dass zumindest einen Effekt, positiv oder negativ, auf einen Organismus, wie einen pflanzlichen, tierischen und/oder menschlichen Körper oder einen Bestandteil davon, hat. Wie oben ausgeführt, ist der Organismus ein therapeutischer Organismus. Das Kombinationspräparat kann auch mehr als eine verabreichbare Einheit haben, wobei zum Beispiel zwei verabreichbare Einheiten zeitlich und/oder räumlich unabhängig voneinander gegeben werden können. Bei einem Kombinationspräparat mit zwei verabreichbaren Einheiten kann je Einheit beispielsweise ein behandlungsrelevanter Bestandteil verabreicht werden.

Ein anti-idiotypisches Antikörperfragment stellt zumindest einen erkennungsrelevanten Anteil eines spezifischen Antikörpers gegen zumindest einen anderen Antikörper, welcher im selben Organismus gebildet wird, dar. Unter dem Begriff "idiotypisches Antikörperfragment" soll die Gesamtheit aller Antikörperfragmente gegen ein bestimmtes Epitop verstanden werden. Dieses kann beispielsweise gegen zumindest einen Teil des Enzyms Neuraminidase sein, welches sich auf der Oberfläche des Newcastle-Disease-Virus befindet und mit dem der Virus an die Wirtszelle und/oder Krebszelle andockt. Als Abwehrreaktion werden im Körper physiologisch gegen zumindest einen Teil dieses Epitops Antikörper aller Immunglobulinklassen (IgM, IgG, IgA und IgE) gebildet. Alle Antikörper und gegebenenfalls auch deren Fragmente, deren gemeinsames Merkmal darin besteht, dass ihr Fab-Anteil zu zumindest einem Teil des Epitops, hier bspw. der Neuraminidase, passt bzw. diesen spezifisch erkennt, sollen als "idiotypische" Antikörperfragmente gegen das Epitop, hier exemplarisch Neuraminidase, verstanden werden. Diese Antikörper werden im folgenden Text als weiteres Antikörperfragment oder als anti-Epitop Antikörperfragment bezeichnet (Näheres siehe unten).

In diesem Zusammenhang soll unter einem Antikörperfragment jedes, vom Fachmann für einsetzbar erachtete Fragment eines Antikörpers, wie beispielsweise ein F(ab')₂-Fragment, Fab'-Fragment, Fab-Fragment oder scFv-Fragment (ein so genanntes single-chain variable fragment), di-scFv, sdAb (ein so genannter single domain antibody), aber auch ein kompletter Antikörper verstanden werden. Grundsätzlich wäre es auch denkbar, als Antikörperfragment einen so genannten trifunctional antibody, ein so genanntes chemically linked F(ab')₂-Fragment oder einen BiTE (ein so genanntes bi-specific T-cell enganger) einzusetzen.

Es kann entweder ein Antikörperfragment oder eine Vielzahl von Antikörperfragmenten eingesetzt werden. Diese könnten vom selben Typ bzw. von selber Herkunft oder von unterschiedlichen Typen bzw. Ursprüngen sein. Es kann also beispielsweise eine Mischung an verschieden aufgebauten Fragmenten eingesetzt werden. Auch wäre es möglich, dass diese Fragmente aus unterschiedlichen Produktionsorganismen stammen (Maus, Kaninchen, Esel etc., weitere Details siehe unten). Zudem könnte(n) das/die anti-idiotypischen Antikörperfragment(e) polyklonalen oder monoklonalen Ursprungs sein. Im folgenden Text wird, unabhängig von Anzahl, Zusammensetzung und genauem Aufbau - also auch ein kompletter Antikörper -, das zumindest eine anti-idiotypische Antikörperfragment generell als das anti-idiotypische Antikörperfragment bezeichnet. Bevorzugt wäre jedoch ein Einsatz von vollständigen Antikörpern, wodurch eine besonders hohe Halbwertszeit des anti-idiotypischen Antikörpers im behandelten Organismus erreicht werden kann.

Unter einem Organismus soll jeder, vom Fachmann für einsetzbar erachtete Organismus, wie beispielsweise ein Bakterium, ein Virus, ein Achaea, ein Pilz, ein Protozoon, eine Alge, ein Parasit, wie bspw. ein Endoparasit (Band-, Spul- oder Fadenwurm) oder ein Ektoparasit, verstanden werden. Es kann entweder ein Organismus oder eine Vielzahl von Organismen eingesetzt werden. Diese könnten vom selben Typ oder von sich unterscheidenden Typen sein. Somit kann auch eine Kombination von unterschiedlichen Organismen zum Einsatz kommen. Im folgenden Text wird, unabhängig von der Anzahl und der Zusammensetzung, der zumindest eine Organismus als der Organismus bezeichnet. Zudem werden das zumindest eine Epitop, der zumindest eine Exzipient und das zumindest eine Vehikel als das Epitop, der Exzipient, das Vehikel bezeichnet.

Auch das weitere anti-Epitop Antikörperfragment soll jedes, vom Fachmann für einsetzbar erachtete und/oder entstehbare Fragment verstanden werden. Insbesondere stellt das weitere Antikörperfragment einen kompletten bzw. vollständigen Antikörper dar, welcher von einem Empfängerorganismus bzw. Patienten gegen den Organismus gebildet wird (vgl. Ausführungen oben). Zu einer Unterscheidung zwischen dem anti-idiotypischen Antikörperfragment und dem weiteren Antikörperfragment wird das letztere, wie oben angeführt, in folgenden Text auch mit anti-Epitop Antikörperfragment bezeichnet.

Ein Exzipient stellt hier einen Hilfsstoff dar, welcher beispielsweise eine Verträglichkeit, eine Aufnahme, eine Resorption, eine Zufuhr (Spritzen, Schlucken etc.), eine Haltbarkeit, eine Viskosität, einen pH-Wert, eine Löslichkeit, eine Salzkonzentration, eine Stabilität, eine Farbe, einen Geschmack und/oder einen Geruch des Kombinationspräparats und/oder des behandlungsrelevanten Bestandteils dessen unterstützt und/oder beeinflusst. Unter einem Vehikel soll insbesondere ein Träger verstanden werden. Unter der Wendung "ein pharmazeutisch verträglicher Exzipient bzw. ein pharmazeutisch verträgliches Vehikel zumindest für das anti-idiotypische Antikörperfragment" soll verstanden werden, dass der Exzipient und/oder das Vehikel dazu vorgesehen ist, zumindest zusammen mit dem anti-idiotypischen Antikörperfragment zugeführt, verabreicht und/oder aufgenommen zu werden. Ferner soll hier und im folgenden Text unter "vorgesehen" speziell ausgestattet, ausgelegt, vorbereitet, ausgebildet und/oder ausgeführt und analog unter "ausgeführt" speziell ausgestattet, ausgelegt, vorbereitet, ausgebildet und/oder vorgesehen verstanden werden.

Bei Bedarf kann das pharmazeutische Kombinationspräparat aus im Wesentlichen anti-idiotypischem Antikörperfragment ein weiteres oder mehrere Mal(e), beispielsweise in regelmäßigen Abständen, wie etwa alle drei Wochen, verabreicht werden. Bei jeder Gabe und/oder Infusion kann sicherheitshalber ein Spiegel an idiotypischen - anti-Epitop - Antikörpern im Behandelten bestimmt werden, um die zu verwendende Dosis an anti-idiotypischen Antikörperfragmenten im Bedarfsfall zu erhöhen.

Das pharmazeutische Kombinationspräparat dient zu einer Unterdrückung einer - insbesondere spezifischen - Immunantwort und zu einer Unterdrückung einer humoralen Immunantwort gegen den therapeutischen Organismus. Hierdurch kann eine Gegenreaktion auf den Organismus effektiv reduziert und vorteilhaft komplett unterdrückt werden. Dadurch hat sich gezeigt, dass sich eine Krankheitstherapie, wie beispielsweise die Virotherapie, vorteilhaft positiv beeinflussen lässt und dies zu deren Erfolg beiträgt.

Des Weiteren wird vorgeschlagen, dass das pharmazeutische Kombinationspräparat zumindest den einen Organismus umfasst, gegen den der variable Teil des weiteren - anti-Epitop - Antikörperfragments gerichtet ist; und mindestens einen pharmazeutisch verträglichen Exzipienten zumindest für den zumindest einen Organismus und/oder mindestens ein pharmazeutisch verträgliches Vehikel zumindest für den zumindest einen Organismus. Hierdurch kann das anti-idiotypische Antikörperfragment schon gleich bei der ersten Gabe des Organismus verabreicht werden. Eine natürliche Produktion idiotypischer anti-Epitop Antikörperfragmente kann dadurch gegen Organismus-Antigene bereits im Keim unterdrückt werden. Dies führt zu einem schnellen Behandlungserfolg.

Ferner muss die Kombinationspräparatgabe so zu Beginn der Verabreichung nur einmal gegeben werden. Dies ist für den Behandelten, wie beispielsweise einen Krebspatienten, besonders schonend. Hierbei wäre die Art der Gabe sowie der Exzipient, das Vehikel und/oder des Trägers des anti-idiotypischen Antikörperfragments und die des Organismus aufeinander abzustimmen. Die Gabe der Substanzen könnte auch zeitlich im Wesentlichen gleich, aber räumlich getrennt voneinander erfolgen (siehe unten), wobei "im Wesentlichen gleich" zumindest an demselben Tag bedeuten soll. Bei Bedarf kann das Kombinationspräparat aus im Wesentlichen anti-idiotypischen Antikörperfragment und Organismus ein weiteres oder mehrere Mal(e), beispielsweise in regelmäßigen Abständen, wie etwa alle drei Wochen, verabreicht werden.

Bezüglich der Wendung "ein pharmazeutisch verträglicher Exzipient bzw. ein pharmazeutisch verträgliches Vehikel zumindest für den Organismus" wird auf die oben ausgeführte Erklärung zum anti-idiotypischen Antikörperfragment verwiesen, die hier analog verstanden werden soll. Der Exzipient und/oder das Vehikel können hier gleich zu dem Exzipienten und/oder Vehikel für das anti-idiotypische Antikörperfragment ausgeführt sein oder diese können unterschiedlich ausgeführt sein. Hierbei sind jeweils die Eigenschaften des anti-idiotypischen Antikörperfragments und des Organismus sowie die Art der Verabreichung ausschlaggebend zur Wahl des jeweiligen Exzipienten und/oder des Vehikels. Diese Merkmale werden vom Fachmann aufgrund seiner Fachkenntnis selbsttätig bestimmt.

Des Weiteren wird vorgeschlagen, dass der Organismus ein Mikroorganismus ist. Wegen einer geringen Größe eines einzelnen Mikroorganismus und damit einem geringen Gabevolumen kann mit einer Zufuhr eine Vielzahl von Organismen verabreicht werden. Unter einem Mikroorganismus soll jeder, vom Fachmann für einsetzbar erachtete Organismus, wie beispielsweise ein Bakterium, ein Virus, ein Achaea, ein Pilz, ein Protozoon oder eine Alge verstanden werden. Es kann entweder ein einzelner Mikroorganismus oder eine Vielzahl von Mikroorganismen eingesetzt werden. Ferner kann auch eine Kombination von unterschiedlichen Mikroorganismen zum Einsatz kommen.

Vorteilhafterweise ist der Organismus und/oder der Mikroorganismus ein Virus, wodurch ein Organismus zum Einsatz kommt, der eine schnelle und einfache Vermehrung aufweist sowie in einer großen Stückzahl bei kleinem Applikationsvolumen verabreicht werden kann. Zudem sind Viren beispielsweise kleiner als Bakterien, was sie zu dem Organismus der ersten Wahl macht. Unter einem Virus soll jedes, vom Fachmann für einsetzbar erachtetes Virus verstanden werden. Ferner ist es vorteilhaft, wenn der Organismus und/oder der Mikroorganismus ein Virus aus der Gruppe der onkolytischen Viren ist, wodurch eine Bekämpfung von Tumorerkrankungen, wie bspw. Krebs, effektiv und nachhaltig erfolgen kann.

Bevorzugt ist der Organismus und/oder der Mikroorganismus ein Virus aus der Gruppe bestehend aus einem Adenovirus, einem Herpes-simplex-Virus (oHSV), einem Reovirus, einem Parvovirus H1, einem Pockenvirus, einem Masernvirus, einem Sendaivirus, einem Newcastle-Disease-Virus (NDV), einem Picornavirus (pig Enterovirus (PEV), ECHO7-Virus), einem Arbovirus, einem Sindbis-Virus, einem Vesicular stomatitis-Virus (VSV), Myxoma-Virus, einem Aujeszky-Virus (pseudorabies-virus, PRV) und einem Seneca-Valley-Virus. Hierdurch steht eine breite Anzahl an unterschiedlichen Viren bereit, wovon zumindest einer je nach Erkrankungstyp und Progression selektiv eingesetzt werden kann.

Gemäß einer alternativen Realisierung wird vorgeschlagen, dass der Mikroorganismus ein Bakterium ist, wodurch ein Organismus zur Anwendung kommen kann, welcher sich wirtszellunabhängig vermehren kann. Zudem kann eine hohe Anzahl pro kleinem Gabevolumen gegeben werden. Unter einem Bakterium soll jedes, vom Fachmann für einsetzbar erachtetes Bakterium verstanden werden. Bevorzugterweise ist der Organismus und/oder der Mikroorganismus aus der Gruppe der onkolytischen Bakterien. Damit kann ein spezifisches Mittel gegen einen Tumor angewendet werden. Es hat sich hierbei gezeigt, dass eine besonders effektive Bekämpfung stattfindet, wenn der Organismus und/oder der Mikroorganismus ein Bakterium aus der Gruppe bestehend aus Salmonella typhimurium, Clostridium und E. coli ist. Ferner wäre es auch möglich, das Bakterium zu modifizieren oder zu adaptieren.

In einer weiteren Ausgestaltung der Erfindung wird vorgeschlagen, dass das anti-idiotypische Antikörperfragment ein murines Antikörperfragment oder ein chimäres Antikörperfragment ist. Damit kann eine Generierung, eine Gewinnung und eine eventuelle Modifizierung des anti-idiotypischen Antikörperfragments mit gut erforschten sowie routinemäßig angewendeten und damit erfolgversprechenden Verfahren gewonnen werden. Vorteilhaft weist das anti-idiotypische Antikörperfragment hierbei ein hybrides humanes Antikörperfragment oder ein humanisiertes Antikörperfragment auf. Somit kann eine gute Verträglichkeit des anti-idiotypischen Antikörperfragments bei einem Einsatz in einem beispielsweise menschlichen Organismus erzielt werden.

Grundsätzlich kann das anti-idiotypische Antikörperfragment jede Art von chimärem und/oder hybridem Antikörperfragment sein, das sich aus zumindest einem Teil mit Ursprung aus dem Ursprungsorganismus (Organismus, der das anti-idiotypische Antikörperfragment generiert, wie beispielsweise eine Maus, ein Kaninchen, ein Affe, ein Esel, ein Pferd etc.) und zumindest einem Teil, vorbereitet zur Aufnahme in einem Zielorganismus (Organismus, der beispielsweise den Tumor aufweist), zusammensetzt. Hierbei kann der Zielorganismus jeder, vom Fachmann für behandelbar erachteter, tierischer und/oder menschlicher Organismus sein, wie beispielsweise der einer Maus, einer Ratte, eines Hasen, eines Hunds, einer Katze, eines Paarhufers, eines Unpaarhufers (eines Schweins, einer Kuh, eines Pferds, eines Kamel etc.) und insbesondere eines Menschen.

Eine homogene Antikörper-Antikörper-Bindung kann vorteilhaft erreicht werden, wenn das anti-idiotypische Antikörperfragment monoklonalen Ursprungs ist. Hierdurch kann das anti-idiotypische Antikörperfragment in großen Mengen und wiederholt in gleicher Qualität sowie kostengünstig gewonnen werden.

Eine bevorzugte Weiterbildung besteht darin, dass das anti-idiotypische Antikörperfragment ein F(ab')₂-Fragment, ein Fab'-Fragment, ein Fab-Fragment oder scFv-Fragment ist. Durch diese Ausgestaltungen fehlt dem anti-idiotypischen Antikörperfragment der Fc-Anteil, wodurch eine Aktivierung des Komplementsystems im Zielorganismus vorteilhaft unterbleibt. Je kleiner das anti-idiotypische Antikörperfragment ist, umso besser ist eine Gewebegängigkeit im Zielorganismus. Dadurch kann das anti-idiotypische Antikörperfragment gezielt auf seinen Einsatzort abgestimmt ausgewählt werden. Die kurze Halbwertszeit dieser trunkierten anti-idiotypischen Antikörperfragmente kann zum Beispiel durch eine höher frequentierte Gabe ausgeglichen werden.

Es wird zudem vorgeschlagen, dass das Epitop, welches der variable Teil des weiteren - anti-Epitop - Antikörperfragments erkennt, eine Struktur des zumindest einen Organismus und/oder Mikroorganismus ist, welche dazu ausgebildet ist, an eine Wirtszelle anzudocken. Damit kann erreicht werden, dass der Organismus, wie beispielsweise ein Virus, in und/oder aus der Wirtszelle ein- und/oder ausgeschleust werden kann, da er nicht von dem gegen zumindest eines seiner Epitope gerichteten Antikörperfragment abgefangen und somit neutralisiert werden kann.

In diesem Zusammenhang soll unter einem Epitop des Organismus jede, vom Fachmann für sinnvoll erachtete Struktur des Organismus, wie ein Peptid, ein Protein, wie beispielsweise ein Enzym, ein Zuckermolekül, ein Nukleotid, ein Polynukleotid etc. und/oder zumindest ein Teil der genannten Moleküle, verstanden werden. Bevorzugt ist diese Struktur auf einer Oberfläche bzw. einer Außenoberfläche des Organismus angeordnet bzw. in zumindest dem Zustand des Andockens des Organismus an der Wirtszelle auf der Außenoberfläche des Organismus präsentiert. Besonders bevorzugt ist das Epitop zumindest ein Teil eines Membranproteins des Organismus. Ist der Organismus beispielsweise ein Virus, kann das Epitop zumindest ein Teil von Haemagglutinin und/oder Neuraminidase sein. Eine Wirtszelle stellt hier insbesondere eine entartete Wirtszelle also eine Tumorzelle und/oder Krebszelle dar.

Ferner wird vorgeschlagen, dass das anti-idiotypische Antikörperfragment und der pharmazeutisch verträgliche Exzipient zumindest für das zumindest eine anti-idiotypische Antikörperfragment und/oder das mindestens eine pharmazeutisch verträgliche Vehikel zumindest für das zumindest eine anti-idiotypische Antikörperfragment, sowie der zumindest eine Organismus und der mindestens eine pharmazeutisch verträgliche Exzipient zumindest für den zumindest einen Organismus und/oder das mindestens eine pharmazeutisch verträgliche Vehikel zumindest für den zumindest einen Organismus, jeweils so ausgeführt sind, dass sie räumlich und/oder zeitlich unabhängig voneinander verabreichbar sind.

In anderen Worten, das anti-idiotypische Antikörperfragment und sein Exzipient, Vehikel oder Träger sowie der Organismus und sein Exzipient, Vehikel oder Träger sind jeweils so ausgeführt, dass sie räumlich und/oder zeitlich unabhängig voneinander verabreichbar sind. Damit kann sowohl das anti-idiotypische Antikörperfragment wie auch der Organismus je nach Bedarf verabreicht werden. Zudem können der Exzipient, das Vehikel oder der Träger wie auch andere Eigenschaften, wie beispielsweise Dosis, Temperatur, Verabreichungsvolumen, Aufnahmekinetik, Verträglichkeit etc., so jeweils auf das anti-idiotypische Antikörperfragment und/oder den Organismus abgestimmt werden.

Es bestünde z. B. die Möglichkeit den Organismus zu dessen besserer Zugänglichkeit räumlich in der Nähe der Wirtszelle zu verabreichen und das anti-idiotypische Antikörperfragment zum Unterdrücken und/oder Abfangen der humoralen Immunantwort des Behandelten gegen den therapeutischen Organismus in die Blutbahn zu geben. Unter "räumlich unabhängig" soll verstanden werden, dass die Gabe der Substanzen entweder auf unterschiedliche Art, wie bspw. i.a. (intraarteriell, intraartikulär), i.c., i.m., i.o., i.p., i.t., i.th., intrakardial, i.v., intravitreal, s.c. s.l. oder peroral, oder auf gleiche Art aber an unterschiedlichen Körperstellen (z. B. Arterie/Vene rechter Arm und Arterie/Vene linker Arm; s.c. Bauchhaut und s.c. Gesäß usw.) erfolgt.

Zu dem Verständnis von "zeitlich unabhängig" kann auf die Erklärung oben verwiesen werden, die hier analog verstanden werden soll. Es wäre beispielsweise denkbar, dass das anti-idiotypische Antikörperfragment zeitlich vor der Gabe des Organismus verabreicht wird. Damit wirkt das anti-idiotypische Antikörperfragment als Prophylaxe, wodurch die natürliche Produktion von anti-Epitop Antikörperfragmenten gegen Virusantigene bereits im Keim unterdrückt werden kann. Um eine Überlastung des behandelten Organismus zu verhindern, ist es beispielsweise auch möglich, zuerst den Organismus zu verabreichen und zeitlich danach erst das anti-idiotypische Antikörperfragment. Der Zeitversatz könnte sich zum Beispiel an einer erfahrungsgemäßen Entstehung von anti-Epitop Antikörperfragmenten orientieren.

Denkbar wäre auch, zur Aufrechterhaltung einer effektiv benötigten Menge an anti-idiotypischem Antikörperfragment im Organismus, das anti-idiotypische Antikörperfragment zeitlich nach einer gemeinsamen Gabe von anti-idiotypischem Antikörperfragment und Organismus einmalig oder mehrmalig, insbesondere in gleichmäßigen Zeitabständen, zu geben oder aufzufrischen. Die Notwendigkeit der Gabe und/oder deren Dosis könnte auch durch eine Bestimmung des Serumspiegel an idiotypischen - anti-Epitop - Antikörperfragmenten und/oder an anti-idiotypischen Antikörperfragmenten und/oder an Organismus im Behandelten ermittelt werden.

Eine weitere Ausgestaltung der Erfindung sieht vor, dass das anti-idiotypische Antikörperfragment so ausgeführt ist, dass es zum Reduzieren und insbesondere zum Neutralisieren einer inhibierenden Aktivität des Weiteren - anti-Epitop - Antikörperfragments vorbereitet ist. Damit kann effektiv und zuverlässig die humorale Immunantwort des Behandelten gegen den therapeutischen Organismus unterdrückt werden.

Günstigerweise ist das anti-idiotypische Antikörperfragment so ausgeführt, dass es zum Reduzieren und insbesondere zum Neutralisieren der inhibierenden Aktivität eines Antikörperfragments gerichtet gegen zumindest ein Epitop eines onkolytischen Virus vorbereitet ist. Hierdurch kann gewährleistet werden, dass der Virus seine Zielzelle, Wirtszelle und/oder Krebszelle erreicht, an diese andocken kann und diese lysiert. Das Epitop ist bevorzugt ein Teil der Neuraminidase beispielsweise eines Newcastle-Disease-Virus. Somit kann ein System zum Einsatz kommen, dessen Charakteristika bekannt sind und das sich zur Krebsbekämpfung schon vielfach bewährt hat.

Des Weiteren kann es vorteilhaft sein, wenn das anti-idiotypische Antikörperfragment so ausgeführt ist, dass es zum zumindest teilweisen Ersetzen von Zytostatika vorbereitet ist. Damit können Nebenwirkungen einer Behandlung bzw. einer Krebstherapie für den Behandelten vorteilhaft reduziert oder sogar verhindert werden. Auch können Behandlungskosten verringert werden. Generell kann die Behandlung trotzdem durch weitere Medikamente, Hilfspräparate oder Anwendungen unterstützt und/oder ergänzt werden.

Zweckmäßigerweise ist der Organismus und/oder der Mikroorganismus so ausgewählt, dass er zum Infizieren zumindest einer entarteten Wirtszelle und/oder Krebszelle vorbereitet ist. Hierdurch kann mit einer Wahl von Merkmalen des Organismus die Behandlung zielgenau ausgerichtet werden, was der Behandlung ein großes Erfolgspotential gibt. Dies Vorbereitung kann durch die Wahl eines geeigneten Organismus erfolgen oder durch eine gezielte Modifikation des Organismus abgestimmt auf den speziellen Behandlungsbedarf.

Ferner ist es vorteilhaft, wenn der Organismus und/oder der Mikroorganismus ein onkolytisches Virus ist, mit welchem die zumindest eine entartete Wirtszelle infizierbar ist. Diese Virengruppe zeigt eine besonders hohe Infektions- und Erfolgsquote. Es hat sich gezeigt, dass insbesondere das Newcastle-Disease-Virus, ein Reovirus oder ein Vesicular stomatitis-Virus (VSV) eine gute Wirkung erzielt.

Des Weiteren geht die Erfindung aus von einem pharmazeutischen Kombinationspräparat, zu einer Unterdrückung humoralen Immunantwort gegen einen therapeutischen Organismus, enthaltend: Zumindest ein anti-idiotypisches Antikörperfragment mit zumindest einer Erkennungsstelle, welche spezifisch zumindest eine Struktur eines variablen Teils eines weiteren Antikörperfragments erkennt, wobei der variable Teil des weiteren Antikörperfragments so ausgeführt ist, dass er zum Erkennen von zumindest einem Epitop zumindest des Organismus, insbesondere eines Mikroorganismus, vorbereitet ist; zumindest den Organismus, insbesondere den Mikroorganismus, gegen den der variable Teil des weiteren Antikörperfragments gerichtet ist; und mindestens einen pharmazeutisch verträglichen Exzipienten und/oder mindestens ein pharmazeutisch verträgliches Vehikel.

Durch diese erfindungsgemäße Realisierung kann ein Kombinationspräparat zur Verfügung gestellt werden, mit welchem schon gleich bei der ersten Gabe von Organismus auch das anti-idiotypische Antikörperfragment verabreicht werden kann. Dadurch kann vorteilhaft die Immunreaktion des Körpers des Behandelten und somit die natürliche Produktion idiotypischer - anti-Epitop - Antikörperfragmente gegen Organismusantigene bereits im Keim unterdrückt werden. Vorteilhaft ergibt sich dadurch ein schneller Behandlungserfolg. Damit kann eine Gabe von zusätzlich immunmodulierenden und/oder immunsupprimierenden Substanzen reduziert werden oder völlig unterbleiben. Folglich ist eine Behandlung mit dem Kombinationspräparat besonders gut verträglich und weist geringe bis keine Nebenwirkungen auf. In Folge dessen hat es eine besonders hohe Erfolgsquote in der Behandlung von Erkrankungen, bei denen gegen den zur Behandlung eingesetzten Organismus Immunreaktionen des Empfängerorganismus ausgelöst werden, wie beispielsweise im Zusammenhang mit Krebserkrankungen. Damit kann das Kombinationspräparat beispielsweise erfolgreich die Nachfolge der Chemotherapie antreten, deren Möglichkeiten schon seit geraumer Zeit bis zur Grenze der "Menschlichkeit" ausgereizt sind.

Das Kombinationspräparat kann hier einen Exzipienten und/oder ein Vehikel sowohl für das anti-idiotypische Antikörperfragment und den Organismus aufweisen. Hierbei wäre die Art der Gabe sowie der Exzipient, das Vehikel und/oder des Trägers des anti-idiotypischen Antikörperfragments und die des Organismus aufeinander abzustimmen. Es wäre aber auch denkbar, dass das Kombinationspräparat hier einen Exzipienten und/oder ein Vehikel für das anti-idiotypische Antikörperfragment und einen Exzipienten und/oder ein Vehikel für den Organismus aufweist. Ferner kann das Kombinationspräparat aus im Wesentlichen anti-idiotypischem Antikörperfragment und Organismus in zwei oder mehreren Einheiten verabreicht werden und zwar räumlich und/oder zeitlich unabhängig voneinander.

Zudem geht die Erfindung aus von einem pharmazeutischen Kombinationspräparat zu einer Unterdrückung einer humoralen Immunantwort gegen einen therapeutischen Organismus, enthaltend: Zumindest ein anti-idiotypisches Antikörperfragment mit zumindest einer Erkennungsstelle, welche spezifisch zumindest eine Struktur eines variablen Teils eines weiteren Antikörperfragments erkennt, wobei der variable Teil des weiteren Antikörperfragments so ausgeführt ist, dass er zum Erkennen von zumindest einem Epitop, insbesondere Neuraminidase, eines onkolytischen Virus, insbesondere New-Castle-Disease Virus, Reovirus oder VSV, vorbereitet ist; zumindest das eine onkolytische Virus, insbesondere New-Castle-Disease Virus, Reovirus oder VSV, gegen den der variable Teil des weiteren Antikörperfragments gerichtet ist; und mindestens einen pharmazeutisch verträglichen Exzipienten und/oder mindestens ein pharmazeutisch verträgliches Vehikel.

Durch die erfindungsgemäße Ausgestaltung kann ein Kombinationspräparat zur Verfügung gestellt werden, mit welchem die Krebsbehandlung bzw. die Virotherapie mit einem Virus durchgeführt werden kann, das gute Erfolge erzielt. Ein solches Präparat ist besonders gut verträglich und weist geringe bis keine Nebenwirkungen auf. In Folge dessen hat es eine besonders hohe Erfolgsquote in einer Behandlung von Erkrankungen, bei denen gegen den zur Behandlung eingesetzten Organismus Immunreaktionen des Empfängerorganismus ausgelöst werden, wie beispielsweise im Zusammenhang mit Krebserkrankungen. Erst durch die zusätzliche Gabe anti-idiotypischer Antikörperfragmente wird die Virotherapie einen entscheidenden Durchbruch erzielen, um die Nachfolge der Chemotherapie antreten zu können, deren Möglichkeiten bis zur Grenze der "Menschlichkeit" ausgereizt sind. Die Ausführungen, die oben zum Exzipienten und/oder Vehikel getroffen wurden, sind hier analog anzuwenden.

Ferner geht die Erfindung aus von einem anti-idiotypischen Antikörperfragment mit zumindest einer Erkennungsstelle, welche spezifisch zumindest eine Struktur eines variablen Teils eines weiteren Antikörperfragments erkennt, wobei der variable Teil des weiteren Antikörperfragments so ausgeführt ist, dass er zum Erkennen von zumindest einem Epitop zumindest eines therapeutischen Organismus, insbesondere eines Mikroorganismus, bevorzugt eines onkolytischen Virus, besonders bevorzugt ein New-Castle-Disease Virus, eines Reovirus oder VSV, vorbereitet ist, zur Verwendung als Medikament.

Durch die erfindungsgemäße Realisierung kann ein anti-idiotypisches Antikörperfragment zur Verfügung gestellt werden, welches zum Behandeln einer Tumorerkrankung effektiv eingesetzt werden kann. Wie oben beschrieben, werden vom Körper des Patienten während der Behandlung mit Viren Antikörper gegen Virusepitope gebildet. Hierdurch schlägt die Virotherapie fehl. Obwohl es also grundsätzlich eine physiologische Gegenmaßnahme gibt, nämlich die Bildung von anti-idiotypischen Antikörpern gegen die Antikörper gegen die Virusepitope, unterbleibt diese physiologische Gegenregulation, solange Viren zugeführt werden. Folglich kann der Körper während der krebstherapierenden Behandlung nicht auf die Ursache, welche die Virotherapie ausbremst, reagieren. Demgemäß ist es von großem Vorteil, wenn diese Gegenregulation auf medikamentöser Weise von außen erfolgt.

Das anti-idiotypische Antikörperfragment wird durch eine gezielte Produktion im Labor erhalten. Es wird also aufgrund eines von außen künstlich zugeführten Reizes unter Laborbedingungen gebildet. Das anti-idiotypische Antikörperfragment dient zu einer prophylaktischen und/oder therapeutischen Zufuhr von außen.

Eine Gewinnung des anti-idiotypischen Antikörperfragments kann beispielsweise folgendermaßen erfolgen. Aus Serum zumindest eines Patienten, der über einen längeren Zeitraum, wie beispielsweise drei Monate, einen bestimmten Organismus und/oder Mikroorganismus, wie z. B. ein onkolytisches Virus, erhalten *hat, wird zumindest ein idiotypisches - anti-Epitop - Antikörperfragment extrahiert. Dieses wird nachfolgend in zumindest ein Labortier, wie beispielsweise einer Maus, injiziert, um diese zur Bildung anti-idiotypischer Antikörper zu stimulieren. Diese Tierantikörper können dann wahlweise "humanisiert", also für eine Anwendung im Menschen, angepasst werden. Dies erfolgt, indem einzelne Domänen, wie beispielsweise ein Fc-Teil oder eine variable Region, gezielt verändert oder "menschlicher gemacht" werden. Dies beinhaltet natürlich nicht die Erkennungsstelle, welche die Struktur des Antikörperfragments gegen das Epitop des Organismus erkennt. Dadurch kann die Verträglichkeit des anti-idiotypischen Antikörperfragments entscheidend erhöht werden. Es hat sich gezeigt, dass selbst bei jahrelanger Zuführung Anaphylaxien selten sind. Diese "Humanisierung" könnte beispielsweise analog zur Gewinnung des monoklonalen Antikörpers Trastuzumab (Herceptin®) geschehen. Alternativ und/oder zusätzlich könnte bei Bedarf der Tierantikörper durch spezifischen Verdau modifiziert werden, wodurch zumindest eines der oben genannten trunkierten anti-idiotypischen Antikörperfragmente erhalten werden kann. Ein solches Fragment könnte ferner weiter modifiziert werden. Grundsätzlich könnte der Tierantikörper auch für eine Anwendung bei einer anderen Tierspezies angepasst bzw. modifiziert werden.

Zusätzlich geht die Erfindung aus von einem Polynukleotid codierend das erfindungsgemäße anti-idiotypische Antikörperfragment. Das Polynukleotid kann jedes, vom Fachmann für einsetzbar erachtete Polynukleotid, wie bspw. DNA oder RNA etc., sein. Das Polynukleotid könnte auch ein zusätzlicher Bestandteil des Kombinationspräparats und/oder ein alternativer Bestandteil des Kombinationspräparats anstelle des anti-idiotypischen Antikörperfragments sein. Dadurch kann eine alternative Zufuhr des anti-idiotypischen Antikörperfragments bereitgestellt werden, die unterschiedliche Kinetiken im Vergleich zu dem anti-idiotypischen Antikörperfragment aufweisen kann. Damit kann das Polynukleotid codierend das anti-idiotypische Antikörperfragment auch zur Verwendung als Medikament dienen.

Ferner könnte das Polynukleotid auch als Anteil eines Vektors, insbesondere eines viralen Vektors, ausgeführt sein, wobei der Vektor als ein Vehikel bzw. ein Träger für das Polynukleotid angesehen werden kann. Beispielsweise könnte die Sequenz des anti-idiotypischen Antikörperfragments so in die Wirtszelle gelangen und ähnlich wie das Virus selbst dort vermehrt werden. Damit könnte eine weitere Zufuhr des anti-idiotypischen Antikörperfragments entfallen, da der Körper des Behandelten diesen selbst produziert. Hierbei könnten weitere Modifikationen des Polynukleotids und/oder des Vektors vorgenommen werden, um ein fehlerfreies Prozessieren zu ermöglichen. Diese Maßnahmen wird der Fachmann selbsttätig aufgrund seiner Fachkenntnis auswählen.

Die oben beschriebenen Eigenschaften, Merkmale und Vorteile dieser Erfindung, sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- FIG 1: eine schematische Darstellung eines Organismus bei einem Andocken an eine Zellmembran einer Wirtszelle,
- FIG 2: ein Diagramm, das eine Aktivität eines Tumormarkers über die Zeit in Abhängigkeit von der Gabe des Mikroorganismus der FIG 1 darstellt,
- FIG 3: eine schematische Darstellung einer Bindung eines anti-Epitop Antikörperfragments an ein Epitop des Organismus der FIG 1,
- FIG 4: eine schematische Darstellung eines erfindungsgemäßen Kombinationspräparats mit dem Organismus der FIG 1 und einem anti-idiotypischen Antikörperfragment als zwei unabhängig voneinander verabreichbare Einheiten,
- FIG 5: eine schematische Darstellung dreier Interaktionssituationen des Organismus der FIG 1, des anti-Epitop Antikörperfragments der FIG 3 und des anti-idiotypischen Antikörperfragments der FIG 4 mit einer Zellmembran einer Wirtszelle,
- FIG 6: ein Diagramm, das eine Aktivität eines Tumormarkers über die Zeit in Abhängigkeit von der Gabe des Mikroorganismus und des anti-idiotypischen Antikörperfragments der FIG 4 darstellt,
- FIG 7: eine schematische Darstellung des anti-idiotypischen Antikörperfragments der FIG 4 mit einer Erkennungsstelle für das anti-Epitop Antikörperfragment der FIG 5,
- FIG 8: eine schematische Darstellung des anti-idiotypischen Antikörperfragments der FIG 4 als chimäres oder als humanisiertes Antikörperfragment,
- FIG 9: eine schematische Darstellung einer Bindung des anti-idiotypischen Antikörperfragments der FIG 7 an das anti-Epitop Antikörperfragment der FIG 5,
- FIG 10: eine schematische Darstellung eines ersten alternativen anti-idiotypischen Antikörperfragments als ein F(ab')₂-Fragment,
- FIG 11: eine schematische Darstellung eines zweiten alternativen anti-idiotypischen Antikörperfragments als ein Fab'-Fragment,
- FIG 12: eine schematische Darstellung eines dritten alternativen anti-idiotypischen Antikörperfragments als ein Fab-Fragment,
- FIG 13: eine schematische Darstellung eines vierten alternativen anti-idiotypischen Antikörperfragments als ein scFv-Fragment,
- FIG 14: eine schematische Darstellung eines ersten alternativen pharmazeutischen Kombinationspräparats mit dem Organismus der FIG 1 und dem anti-idiotypischen Antikörperfragment der FIG 5 als eine verabreichbare Einheit und
- FIG 15: eine schematische Darstellung eines zweiten alternativen pharmazeutischen Kombinationspräparats mit dem Organismus der FIG 1 und einem Vektor, der das anti-idiotypische Antikörperfragment der FIG 5 codiert.

FIG 1 zeigt in einer schematischen Darstellung einen Körper 40 eines Behandelten, wie beispielsweise einen menschlichen Krebspatienten, mit einem bösartigen Tumor 42, wie z. B. einem Pankreastumor (die Dimensionen sind in FIG 1 nicht wahrheitsgemäß wiedergegeben). Als momentan gängige Therapie kann in einem fortgeschrittenen Krankheitsstadium die sogenannten Virotherapie angewendet werden. Hierbei wird dem Körper 40 des Patienten beispielsweise i.v. und/oder i.a. ein Organismus 24 bzw. eine Vielzahl von Organismen 24 injiziert (Details siehe unten zu FIG 2). Der Organismus 24 stellt einen Mikroorganismus 30, in der Form eines onkolytischen Virus 32, wie beispielsweise eines Newcastle-Disease-Virus 32, dar. Ferner ist der Organismus 24 ein therapeutischer Organismus 24. Das Virus 32 dringt in eine entartete Wirtszelle 34 bzw. eine Tumorzelle des Körpers 40 ein und kann sich in der Wirtszelle 34 vermehren. Hierfür weist das Virus 32 an seiner Oberfläche 44 verschiedene Membranproteine 46, wie beispielsweise Neuraminidase 36 und Haemagglutinin 48 auf. Um in die Wirtszelle 34 einzudringen, dockt ein Teil der Neuraminidase 36 an einen Rezeptor 50 einer Zellmembran 52 der Wirtszelle 34 an. Das Virus 32 vermag letztlich die Wirtszelle 34 bzw. die Tumorzelle zu lysieren, was diese beseitigt. Damit kann die Krankheitsprogression entscheidend verlangsamt werden.

Hierbei hat sich gezeigt, dass die Virotherapie am Anfang gut anschlägt, wenn beispielsweise in den ersten sechs Behandlungstagen mehrere Male Virus 32 i.v. injiziert wird (siehe erste Gruppe an Pfeilen in FIG 2). Dies ist in FIG 2 zu sehen (siehe Kurvenverlauf Tage 1 bis 6), die ein Diagramm zeigt, bei dem eine Aktivität eines Tumormarkers CA 19-9 in IU/ml über die Zeit in Abhängigkeit von der Gabe des Virus 32 dargestellt ist. Danach zeigt sich ein rapider Anstieg des Tumormarkers CA 19-9, wie dies am Kurvenverlauf des Zeitraums zwischen Tag 8 und 19 zu sehen ist. Wird nun erneut Virus 32 injiziert (vgl. zweite Gruppe an Pfeilen), dieses Mal zusätzlich zu der i.v. Injektion auch als i.a Injektion, kann wieder eine Abnahme des Tumormarkers CA 19-9 beobachtet werden (vgl. Kurvenverlauf des Zeitraums zwischen Tag 19 und 37). Nach Tag 37 steigt der Tumormarker CA 19-9 wieder rasant und stetig an (vgl. Kurvenverlauf des Zeitraums zwischen Tag 37 und 51). Auch eine weitere Virusgabe kann die Krankheitsprogression nicht mehr aufhalten (vgl. dritte Gruppe an Pfeilen des Zeitraums zwischen Tag 39 und 51).

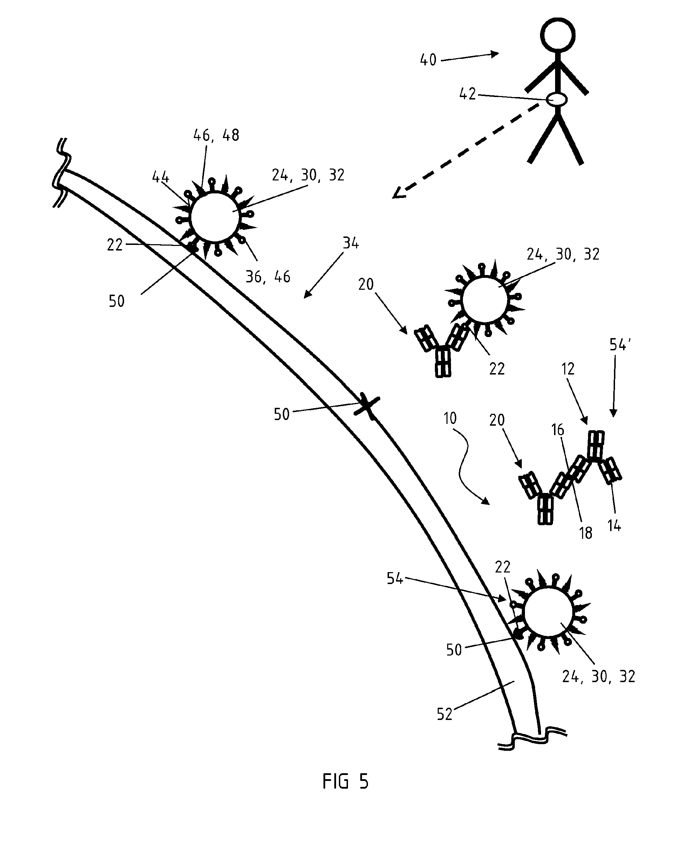

Es wird davon ausgegangen, dass das Immunsystem des Krebspatienten auch die heilsamen Mikroorganismen 30 bzw. Viren 32 als Feinde betrachtet und dagegen das zelluläre wie das humorale Abwehrsystem durch Bildung von zytotoxischen T-Zellen und Antikörpern, im folgenden Text als Antikörperfragment 20 bezeichnet, mobilisiert. Diese gebildeten Antikörperfragmente 20 gegen onkolytische Viren 32 fangen einen großen Teil der Viren 32 ab, bevor diese die Wirtszelle 34 bzw. die Tumorzelle erreichen können. Dies ist schematisch in FIG 3 gezeigt. Das Antikörperfragment 20 bindet mit seinem variablen Teil 18 an ein Epitop 22 des Virus 32, wie beispielsweise der Neuraminidase 36. Damit kann das Antikörperfragment 20 als ein anti-Epitop Antikörperfragment 20 bezeichnet werden. Dadurch kann dieses Molekül der Neuraminidase 36 den Rezeptor 50 der Zellmembran 52 der Wirtszelle 34 nicht mehr binden (vgl. auch FIG 5, zweite Interaktionssituation) und das Virus 32 kann nicht mehr in die Wirtszelle 34 eindringen. Es kann natürlich zu einer Vielzahl von Bindungen zwischen mehreren Antikörperfragmenten 20 und einem Virus 32 kommen. Zur besseren Darstellbarkeit ist lediglich eine Interaktion gezeigt. Eine Lyse und damit eine Bekämpfung der Tumorzellen unterbleiben, wodurch die Virotherapie fehlschlägt.

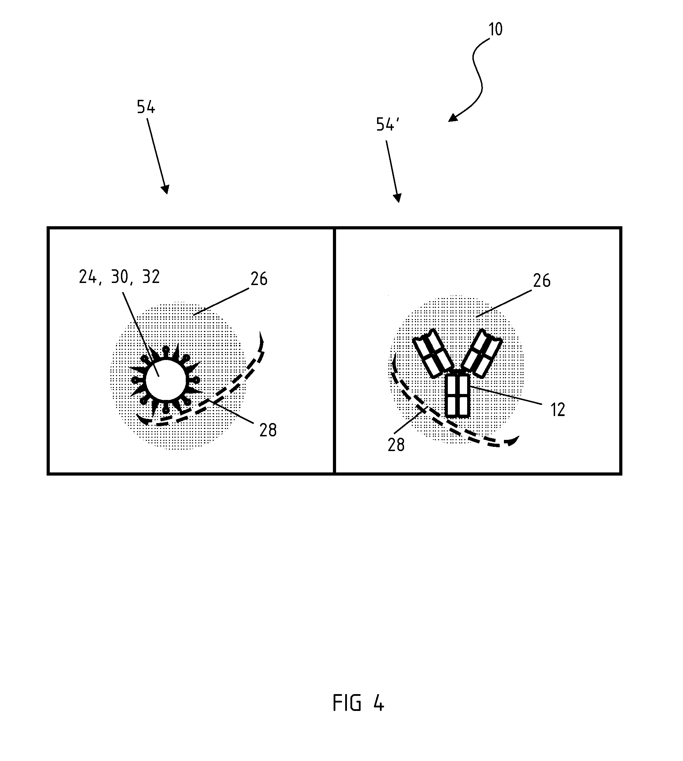

In FIG 4 ist in einer schematischen Darstellung ein erfindungsgemäßes pharmazeutisches Kombinationspräparat 10 zu einer Unterdrückung einer humoralen Immunantwort eines Körpers 40 eines Krebspatienten mit beispielsweise einem fortgeschrittenen Tumor 42 bzw. Pankreaskarzinom und pulmonaler Metastasierung gegen einen therapeutischen Organismus 24 als Reaktion auf eine oben beschriebene Virotherapie gezeigt (vgl. auch FIG 5, Dimensionen nicht wahrheitsgemäß gezeigt).

Bei dieser Therapie wird dem Körper 40 des Patienten beispielsweise i.v. und/oder i.a. ein Organismus 24 bzw. eine Vielzahl von Organismen 24 als eine verabreichbare Einheit 54 injiziert (zur besseren Darstellbarkeit ist lediglich ein Virus 32 gezeigt). Hierbei ist der Organismus 24 so ausgewählt, dass er zum Infizieren einer entarteten Wirtszelle 34 vorbereitet ist. Der Organismus 24 ist ein Mikroorganismus 30 und insbesondere ein onkolytisches Virus 32, mit welchem die entartete Wirtszelle 34 infizierbar ist (Detail siehe Beschreibung zu FIG 1). Das onkolytische Virus 32 ist beispielsweise ausgewählt aus der Gruppe bestehend aus einem Adenovirus, einem Herpes-simplex-Virus (oHSV), einem Reovirus, einem Parvovirus (H1), einem Pockenvirus, einem Masernvirus, einem Sendaivirus, einem Newcastle-Disease-Virus (NDV), einem Picornavirus (pig Enterovirus (PEV), ECHO7-Virus), einem Arbovirus, einem Sindbis-Virus, einem Vesicular stomatitis-Virus (VSV), Myxoma-Virus, einem Aujeszky-Virus (pseudorabies-virus, PRV) und einem Seneca-Valley-Virus. All diese Viren 32 haben Potential in der Krebstherapie gezeigt. Exemplarisch wird hier der Einsatz des Newcastle-Disease-Virus 32 (NDV) beschrieben.

Zudem weist das Kombinationspräparat 10 einen pharmazeutisch verträglichen Exzipienten 26 für den Organismus 24 bzw. die Newcastle-Disease-Viren 32 auf. Wahlweise enthält das Kombinationspräparat 10 ein pharmazeutisch verträgliches Vehikel 28 für die Newcastle-Disease-Viren 32 (vgl. gestrichelt gezeichneter Halbmond).

Anhand FIG 5, erste Interaktionssituation, ist zu sehen, dass ein Teil eines Membranproteins 46, wie beispielsweise ein Molekül Neuraminidase 36, das auf einer Oberfläche 44 des Virus 32 präsentiert wird, an einen Rezeptor 50 einer Zellmembran 52 der Wirtszelle 34 andockt, um in diese einzudringen (vgl. auch Beschreibung zu FIG 1).

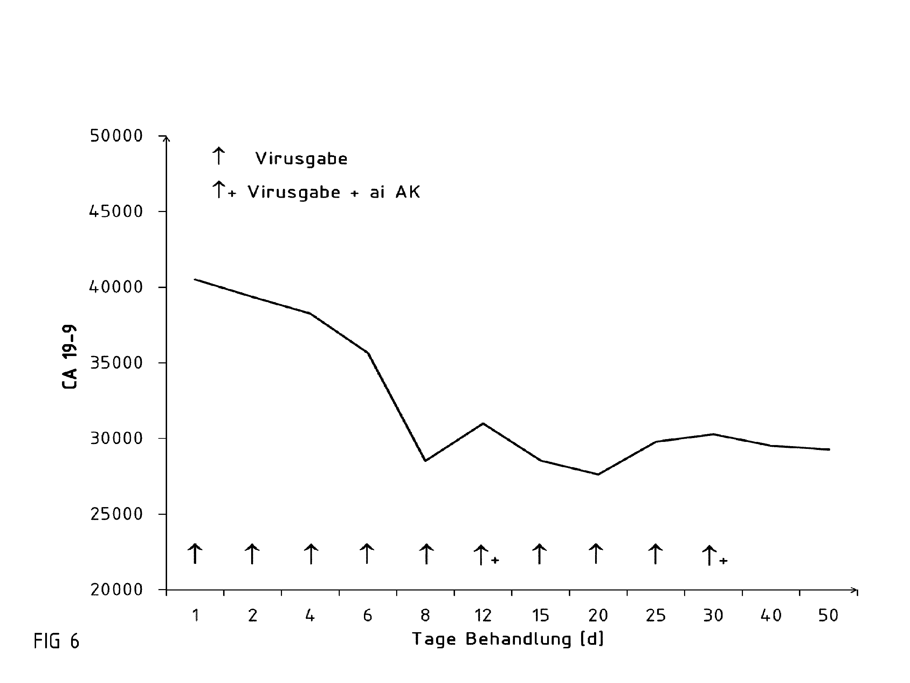

Wie oben beschrieben, konnte gefunden werden, dass die Virotherapie am Anfang der Virengabe gut anschlägt, was sich an der Abnahme eines Tumormarkers CA 19-9 zeigt. Dies ist am Kurvenverlauf repräsentierend die ersten sechs Behandlungstage in FIG 6 zu sehen, wobei FIG 6 ein Diagramm zeigt, bei dem eine Aktivität des Tumormarkers CA 19-9 über die Zeit in Abhängigkeit von der Gabe des Virus 32 und der Gabe eines anti-idiotypischen Antikörperfragments 12 (Details siehe unten) dargestellt ist. Hier wurde beispielsweise in diesen ersten sechs Behandlungstagen mehrere Male Virus 32 i.v. injiziert (siehe die ersten vier Pfeile in FIG 6). Danach zeigt sich ein Anstieg des Tumormarkers CA 19-9, wie dies am Kurvenverlauf des Zeitraums zwischen Tag 8 und 12 zu sehen ist.

Dies wird, wie oben erläutert, darauf zurückgeführt, dass der Körper 40 des Krebspatienten Antikörper, im folgenden Text als anti-Epitop Antikörperfragment 20 bezeichnet, bildet. Hierbei ist der variable Teil 18 des anti-Epitop Antikörperfragments 20 so ausgeführt, dass er zum Erkennen von zumindest einem Epitop 22, wie beispielsweise dem Membranprotein 46 Neuraminidase 36 vorbereitet ist (vgl. FIG 3 und FIG 5, zweite Interaktionssituation). Dieses Epitop 22 ist eine Struktur des Organismus 24, welche dazu ausgelegt ist, an die entartete Wirtszelle 34 anzudocken (vgl. FIG 5, erste und dritte Interaktionssituation). Das anti-Epitop Antikörperfragment 20 bindet also mit seinem variablen Teil 18 an das Epitop 22 bzw. die Neuraminidase 36, wodurch dieses Molekül der Neuraminidase 36 den Rezeptor 50 an der Zellmembran 52 der Wirtszelle 34 des Körpers 40 nicht mehr binden kann (vgl. auch FIG 5, zweite Interaktionssituation). Folglich kann das Virus 32 nicht mehr in die Wirtszelle 34 gelangen und die heilungsfördernde Lyse der Tumorzellen unterbleibt.

Um diese humorale Immunantwort des Körpers 40 gegen den therapeutischen Organismus 24 bzw. das Abfangen des Virus 32 durch das anti-Epitop Antikörperfragment 20 zu unterdrücken, weist das pharmazeutische Kombinationspräparat 10 eine weitere bzw. eine zweite verabreichbare Einheit 54' auf, die zeitlich nach der ersten verabreichbaren Einheit 54 und damit unabhängig von dieser verabreicht wird. Dafür weist das pharmazeutische Kombinationspräparat 10 ein anti-idiotypisches Antikörperfragment 12 bzw. eine Vielzahl von anti-idiotypischen Antikörperfragmenten 12 auf, das/die i.v. und/oder i.a. injiziert wird/werden (zur besseren Darstellbarkeit ist lediglich ein Antikörperfragment 12 gezeigt). Zudem weist das Kombinationspräparat 10 einen pharmazeutisch verträglichen Exzipienten 26 für die anti-idiotypischen Antikörperfragmente 12 auf. Alternativ enthält das Kombinationspräparat 10 ein pharmazeutisch verträgliches Vehikel 28 für die anti-idiotypischen Antikörperfragmente 12 (vgl. gestrichelt gezeichneter Halbmond).

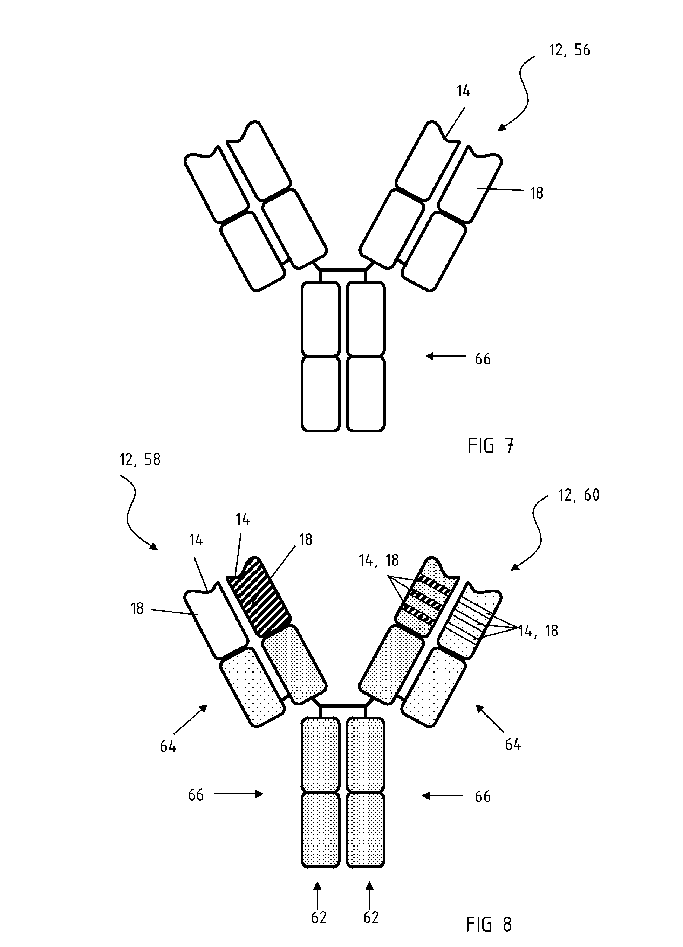

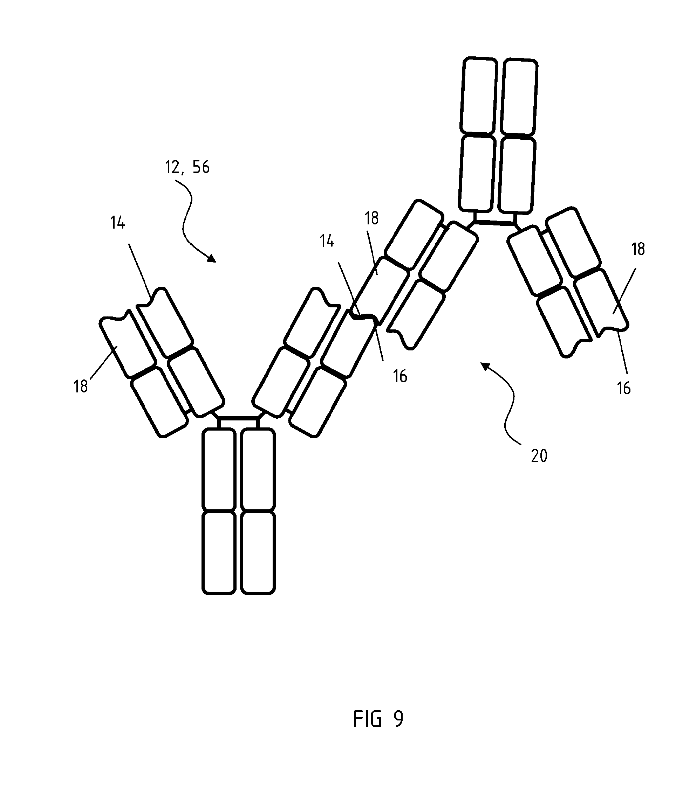

Das anti-idiotypische Antikörperfragment 12, das in FIG 7 im Detail gezeigt ist, umfasst an seinem variablen Teil 18 zumindest eine Erkennungsstelle 14, welche spezifisch zumindest das weitere anti-Epitop Antikörperfragment 20 erkennt. Damit kann die Erkennungsstelle 14 des anti-idiotypischen Antikörperfragments 12 die Struktur 16 des variablen Teils 18 des anti-Epitop Antikörperfragments 20 binden, wie dies FIG 9 darstellt.

Das anti-idiotypische Antikörperfragment 12 stellt einen Antikörper 56 dar (vgl. FIG 7), der in einem Ursprungsorganismus (nicht gezeigt), wie einer Maus, unter Laborbedingungen als Reaktion auf zumindest einen (ein) im Körper 40 eines Behandelten produzierten idiotypischen - anti-Epitop - Antikörper(fragment) 20 (vgl. FIG. 5) angereichert und als muriner und monoklonaler Antikörper 56 extrahiert wurde, wodurch das anti-idiotypisches Antikörperfragment 12 monoklonalen Ursprungs ist. Damit sind die offen gezeigten Anteile bzw. Sequenzen des Antikörpers 56 murinen Ursprungs.

Zu eine besseren Verträglichkeit des anti-idiotypischen Antikörperfragments 12 für den nicht murinen Körper 40 bzw. den Menschen kann das anti-idiotypische Antikörperfragment 12 modifiziert werden. Hierbei sind exemplarisch zwei Möglichkeiten in FIG 8 gezeigt. Diese beiden Möglichkeiten sind zu einer Reduktion der Anzahl der Figuren in einem Antikörpermolekül vereint. In Wirklichkeit würde ein solcher jeweiliger Antikörper 58, 60 zwei identische Gruppen aus einer schweren Kette 62 und einer leichten Kette 64 aufweisen.

Der Antikörper 58 stellt ein chimäres Antikörperfragment 12 mit murinen und humanen Domänen dar (vgl. linke Hälfte des Antikörpers in FIG 8). Damit ist das anti-idiotypische Antikörperfragment 12 sowohl ein murines wie auch ein hybrides humanes Antikörperfragment 12. Die offen und gestrichelt gezeigten Anteile bzw. Sequenzen repräsentieren die variablen Teile 18 bzw. Domänen, sogenannte V-Anteile, der schweren Kette 62 (gestrichelt) und der leichten Kette 64 (offen) des Antikörpers 58. Diese sind murinen Ursprungs. Diese variablen Teile 18 verleihen dem Antikörper 58 seine Spezifität, beinhalten also die Erkennungsstellen 14, welche spezifisch die Struktur 16 des variablen Teils 18 des weiteren anti-Epitop Antikörperfragments 20 erkennen (vgl. FIG 9). Die humanen Domänen der schweren Kette 62 und der leichten Kette 64 sind als unterschiedlich dicht gepunktet dargestellt. Die hohe Dichte repräsentiert konstante Domänen, sogenannte CH1-, CH2- und CH3-Anteile, der schweren Kette 62 (unter anderem ein sogenannter Fc-Teil 66 (CH2 und CH3, englisch für "crystallisable fragment") und die geringere Dichte die Domäne der leichten Kette 64, ein sogenannter C-Anteil. Ein solches chimäres anti-idiotypisches Antikörperfragment 12 wird wegen seines hohen murinen Anteils von einem nicht murinen bzw. hier menschlichen Körper 40 eher schlecht angenommen.

Besser verträglich ist der Antikörper 60, der ein humanisiertes Antikörperfragment 12 mit murinen Anteilen und humanen Domänen ist (vgl. rechte Hälfte des Antikörpers in FIG 8). Damit ist das anti-idiotypische Antikörperfragment 12 sowohl ein murines wie auch ein humanisiertes Antikörperfragment 12. Die offen und gestrichelt gezeigten Streifen bzw. Sequenzen sind Antigenbindungsstellen (abgekürzt CDR, englisch für "Complementarity Determining Region") des humanisierten Antikörperfragments 12. Diese repräsentieren die variablen Teile 18 bzw. Domänen der schweren Kette 62 (gestrichelt) und der leichten Kette 64 (offen) des Antikörpers 60. Diese sind murinen Ursprungs. Diese variablen Teile 18 verleihen dem Antikörper 60 seine Spezifität, beinhalten also die Erkennungsstellen 14, welche spezifisch die Struktur 16 des variablen Teils 18 des weiteren anti-Epitop Antikörperfragments 20 erkennen.

Die humanen Domänen der schweren Kette 62 und der leichten Kette 64 sind, wie oben beschrieben, als unterschiedlich dicht gepunktet dargestellt. Die hohe Dichte repräsentiert konstante Domänen der schweren Kette 62 und die geringere Dichte die Domäne der leichten Kette 64.

Wird nun an Tag 12 das anti-idiotypische Antikörperfragment 12 zusammen mit dem Virus 32 dem Körper 40 injiziert (siehe Pfeil mit Plussymbol des Tags 12 in FIG 6), kann die Erkennungsstelle 14 die Struktur 16 des variablen Teils 18 des anti-Epitop Antikörperfragments 20 binden (vgl. FIG 5, dritte Interaktionssituation und FIG 9). Dadurch wird das anti-Epitop Antikörperfragment 20 "gefangen" und die Neuraminidase 36 des Virus 32 kann wieder zu einem Eindringen in die Wirtszelle 34 an den Rezeptor 50 binden (vgl. FIG 5, dritte Interaktionssituation). Hierdurch kann es wieder zu einer Lyse der entarteten Wirtszelle 34 kommen und die Krankheitsprogression kann verlangsamt werden. Dies zeigt sich auch am Kurvenverlauf des Tumormarkers CA 19-9 des Zeitraums zwischen Tag 12 und 20 bzw. Tag 50 in FIG 6 (siehe die letzten vier Pfeile in FIG 6).

Im Anschluss kann bei Bedarf eine regelmäßige Überwachung des Spiegels des Tumormarkers CA 19-9 oder auch des Virus 32, des anti-Epitop Antikörperfragments 20 oder des anti-idiotypischen Antikörperfragments 12 erfolgen. Aufgrund des Ergebnisses kann eine erneute Injektion oder es können mehrere Injektionen mit Virus alleine (siehe Pfeile der Tage 15, 20, 25 in FIG 6), anti-idiotypischem Antikörperfragment 12 alleine (nicht im Detail gezeigt) oder das Virus 32 zusammen mit dem anti-idiotypischen Antikörperfragment 12 erfolgen, wie dies exemplarisch für die Gabe an Tag 30 gezeigt ist (siehe Pfeil mit Plussymbol des Tags 30 in FIG 6). Die genauen Abstände der Substanzgaben ist also bestimmbar und kann individuell je nach Krankheitsverlauf und Patient festlegt werden. Dies wird der Fachmann aufgrund seiner Fachkenntnis selbsttätig erwägen.

Das anti-idiotypische Antikörperfragment 12 ist also so ausgeführt, dass es zum Reduzieren einer inhibierenden Aktivität des weiteren anti-Epitop Antikörperfragments 20 bzw. eines Antikörperfragments 20 gerichtet gegen zumindest ein Epitop 22, wie beispielsweise der Neuraminidase 36, eines onkolytischen Virus 32 bzw. des Newcastle-Disease-Virus, vorbereitet ist. Damit ist das anti-idiotypische Antikörperfragment 12 zur Verwendung als Medikament gedacht. Durch diese zusätzliche Gabe der anti-idiotypischen Antikörperfragmente 12 wird die Virotherapie den entscheidenden Durchbruch erzielen, um die Nachfolge der Chemotherapie antreten zu können. Folglich ist das anti-idiotypische Antikörperfragment 12 so ausgeführt, dass es zum zumindest teilweisen Ersetzen und bevorzugt zum kompletten Ersetzen von Zytostatika vorbereitet ist. Bei Bedarf können natürlich auch hier weitere, die Therapie unterstützende Mittel, Medikamente und/oder Anwendungen, wie beispielsweise Chemotherapeutika (z. B. Adriblastin, Mitomycin, Gemzar usw.), Immunmodulatoren (z. B. Gepon 1/1), Elektrohyperthermie (EHT), eingesetzt werden.

Das Virus 32 und das anti-idiotypische Antikörperfragment 12 können auch räumlich unabhängig voneinander verabreicht werden; das Virus 32 beispielsweise i.a. und das anti-idiotypische Antikörperfragment 12 i.v. oder peroral.

Alternativ könnte das pharmazeutische Kombinationspräparat 10 als den Organismus 24 bzw. einen Mikroorganismus 30 in der Form eines Bakteriums aufweisen. Dieses Bakterium ist aus der Gruppe der onkolytischen Bakterien bzw. aus der Gruppe bestehend aus Salmonella typhimurium und Clostridium (nicht im Detail gezeigt).

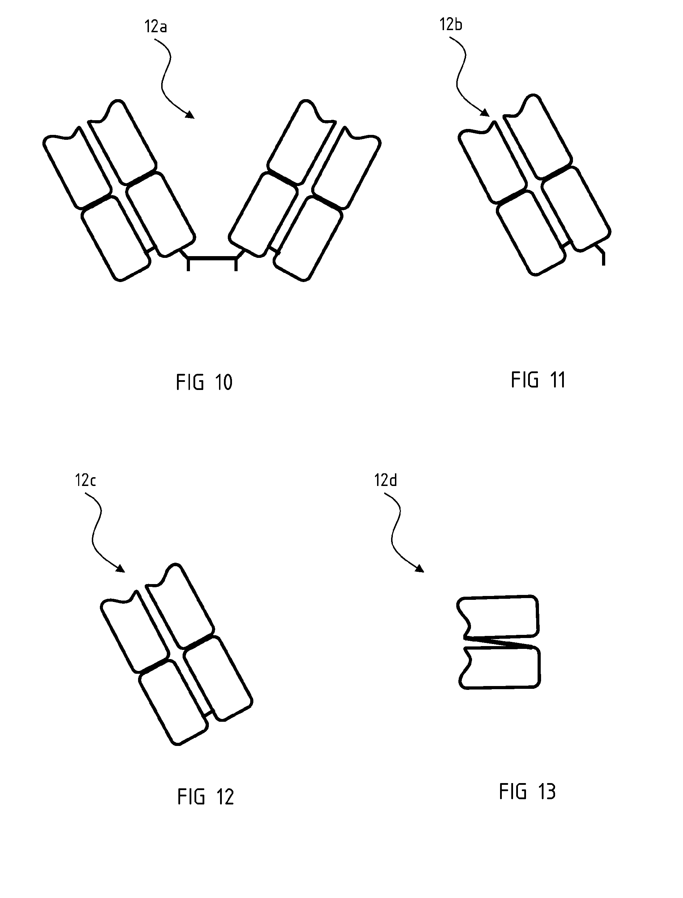

In den FIG 10 bis 15 sind vier alternative anti-idiotypische Antikörperfragmente 12a bis 12d und zwei alternative pharmazeutische Kombinationspräparate 10', 10'' dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele der FIG 10 bis 15 zu dem Ausführungsbeispiel in den FIG 4 bis 9 sind den Bezugszeichen der in den Ausführungsbeispielen der FIG 10 bis 15 voneinander abweichend ausgeführten Bauteile der Buchstaben a bis d bzw. ein oder zwei Apostrophe hinzugefügt. Die nachfolgende Beschreibung beschränkt sich im Wesentlichen auf die Unterschiede zu dem Ausführungsbeispiel in den FIG 4 bis 9, wobei bezüglich gleich bleibender Bauteile, Merkmale und Funktionen auf die Beschreibung des Ausführungsbeispiels in den FIG 4 bis 9 verwiesen werden kann.

In der FIG 10 ist ein erstes alternatives anti-idiotypisches Antikörperfragment 12a gezeigt. Das anti-idiotypische Antikörperfragment 12a unterscheidet sich von dem anti-idiotypischen Antikörperfragment 12 der FIG 4 bis 9 dadurch, dass es ein F(ab')₂-Fragment ist. Dieses wurde aus einem Antikörper 56 generiert (vgl. FIG 7), der in einem Ursprungsorganismus (nicht gezeigt), wie einer Maus, unter Laborbedingungen als Reaktion auf zumindest einen im Körper eines (ein) Behandelten produzierten idiotypischen - anti-Epitop - Antikörper(fragment) (vgl. FIG. 2) angereichert und als muriner und monoklonaler Antikörper 56 extrahiert wurde.

Vor der Gewinnung des F(ab')₂-Fragments wurde der Antikörper 56 zu einem chimären Antikörper 58 oder einem humanisierten Antikörper 60 modifiziert (vgl. FIG 7 und 8, Mustercodierung in FIG 10 nicht gezeigt). Die Gewinnung des F(ab')₂-Fragments könnte beispielsweise durch einen Verdau mit Pepsin erfolgen, bei dem der Fc-Teil 66 des Antikörpers 58, 60 abgespalten wurde (vgl. FIG 8). Alternativ könnte das F(ab')₂-Fragment auch anhand eines allgemein bekannten modifizierten Papain-Verdau des Antikörpers 58, 60 erfolgen.

FIG 11 zeigt ein zweites alternatives anti-idiotypisches Antikörperfragment 12b. Das anti-idiotypische Antikörperfragment 12b unterscheidet sich von dem anti-idiotypischen Antikörperfragment 12 der FIG 4 bis 9 dadurch, dass es ein Fab'-Fragment ist. Dieses wurde aus einem F(ab')₂-Fragment erhalten (vgl. FIG 10). Möglich wäre dies beispielsweise anhand einer Reduktion des F(ab')₂-Fragments mit 2-Mercaptoethyl-amine•HCl (2-MEA), wodurch zwei Fab'-Fragmente erhalten werden. Dieses F(ab')₂-Fragment wiederum wurde beispielsweise aus einem chimären Antikörper 58 oder einem humanisierten Antikörper 60 generiert (vgl. FIG 8, Mustercodierung in FIG 11 nicht gezeigt).

Diese Antikörper 58, 60 wurden aus einem Antikörper 56, der in einem Ursprungsorganismus (nicht gezeigt), wie einer Maus, unter Laborbedingungen als Reaktion auf zumindest einen (ein) im Körper eines Behandelten produzierten idiotypischen - anti-Epitop - Antikörper(fragment) (vgl. FIG. 2) angereichert und als muriner und monoklonaler Antikörper 56 extrahiert (vgl. FIG 7). Die Gewinnung des Fab'-Fragments könnte beispielsweise durch einen Verdau mit Pepsin erfolgen, bei dem ein Fc-Teil 66 des Antikörpers 58, 60 entfernt wurde (vgl. FIG 8). Je Reduktion des F(ab')₂-Fragments können zwei Fab'-Fragmente gewonnen werden. Da das Fab'-Fragment eine freie Thiolgruppe aufweist, kann es wahlweise weiter modifiziert werden. Dies wird der Fachmann aufgrund seiner Fachkenntnis selbsttätig entscheiden.

In der FIG 12 ist ein drittes alternatives anti-idiotypisches Antikörperfragment 12c dargestellt. Das anti-idiotypische Antikörperfragment 12c unterscheidet sich von dem anti-idiotypischen Antikörperfragment 12 der FIG 4 bis 9 dadurch, dass es ein Fab-Fragment ist. Dieses wurde aus einem Antikörper 56 generiert (vgl. FIG 7), der in einem Ursprungsorganismus (nicht gezeigt), wie einer Maus, unter Laborbedingungen als Reaktion auf zumindest einen (ein) im Körper eines Behandelten produzierten idiotypischen - anti-Epitop - Antikörper(fragment) (vgl. FIG. 2) angereichert und als muriner und monoklonaler Antikörper 56 extrahiert.

Vor der Gewinnung des Fab-Fragments wurde der Antikörper 56 zu einem chimären Antikörper 58 oder einem humanisierten Antikörper 60 modifiziert (vgl. FIG 7 und 8, Mustercodierung in FIG 12 nicht gezeigt). Die Generierung des Fab-Fragments könnte beispielsweise durch einen Verdau mit Papain erfolgen, bei dem ein Fc-Teil 66 des Antikörpers 58, 60 abgespalten wird (vgl. FIG 8). Dadurch können je Antikörper 58, 60 zwei Fab-Fragmente gewonnen werden.

FIG 13 stellt ein viertes alternatives anti-idiotypisches Antikörperfragment 12d dar. Das anti-idiotypische Antikörperfragment 12d unterscheidet sich von dem anti-idiotypischen Antikörperfragment 12 der FIG 4 bis 9 dadurch, dass es ein scFv-Fragment ist. Dieses wurde mit Hilfe molekularbiologischer Methoden aus einem abgewandelten Antikörper 56 generiert (vgl. FIG 7). Der Antikörper 56 wurde in einem Ursprungsorganismus (nicht gezeigt), wie einer Maus, unter Laborbedingungen als Reaktion auf zumindest einen (ein) im Körper eines Behandelten produzierten idiotypischen - anti-Epitop - Antikörper(fragment) (vgl. FIG. 2) angereichert und als muriner und monoklonaler Antikörper 56 extrahiert.

Vor der Generierung des scFv-Fragments wurde der Antikörper 56 zu einem chimären Antikörper 58 oder einem humanisierten Antikörper 60 modifiziert (vgl. FIG 8, Mustercodierung in FIG 13 nicht gezeigt). Zur Generierung des scFv-Fragments wurden die beiden variablen Teile 18 einer schweren und einer leichten Kette 62, 64 eines chimären Antikörpers 58 oder eines humanisierten Antikörpers 60 (vgl. FIG 8) künstlich im Labor miteinander verbunden. Beide Domänen sind beispielsweise über einen kurzen Linker verbunden, der sich in der Regel aus einer Sequenz der Aminosäuren Glycin und/oder Serin zusammensetzt.

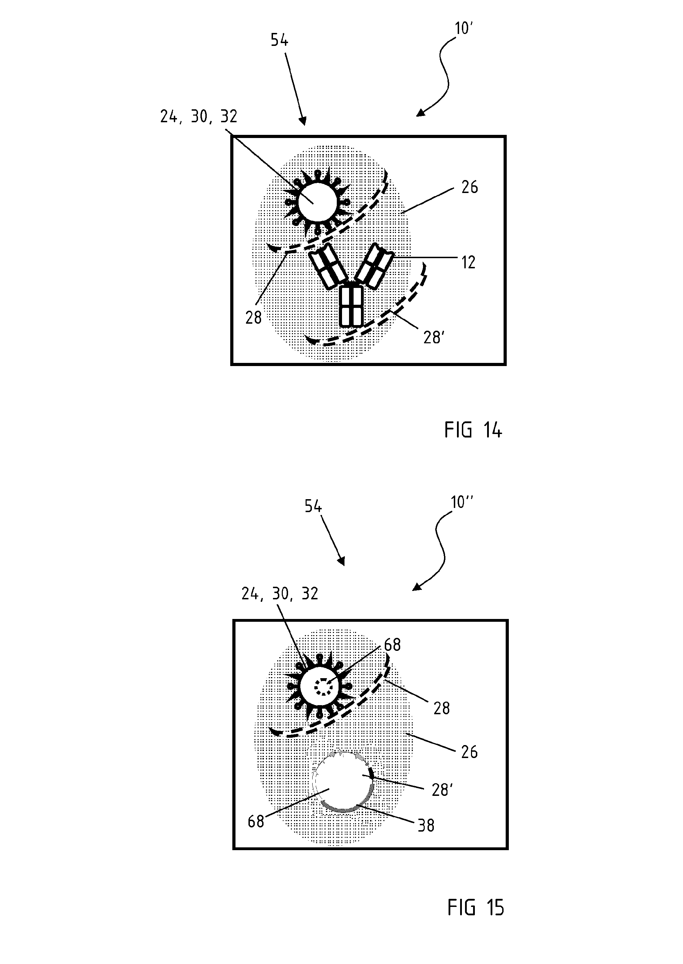

In der FIG 14 ist ein erstes alternatives pharmazeutisches Kombinationspräparat 10' zu einer Unterdrückung einer humoralen Immunantwort gegen einen therapeutischen Organismus 24 gezeigt. Das Kombinationspräparat 10' unterscheidet sich von dem Kombinationspräparat 10 der FIG 4 bis 9 dadurch, dass es eine Vielzahl von anti-idiotypischen Antikörperfragmenten 12 und eine Vielzahl von Organismen 24 bzw. von Mikroorganismen 30, beispielsweise in der Form von Newcastle-Disease-Viren 32, als eine verabreichbare Einheit 54 aufweist (zur besseren Darstellbarkeit ist lediglich ein Antikörperfragment 12 und ein Virus 32 gezeigt). Zudem weist das Kombinationspräparat 10' einen pharmazeutisch verträglichen Exzipienten 26 sowohl für die anti-idiotypischen Antikörperfragmente 12 und die Newcastle-Disease-Viren 32 auf. Wahlweise enthält das Kombinationspräparat 10' ein pharmazeutisch verträgliches Vehikel 28 sowohl für die anti-idiotypischen Antikörperfragmente 12 und die Newcastle-Disease-Viren 32 (vgl. gestrichelt gezeichneter oberer Halbmond). Ferner könnten zwei Vehikel 28, 28' vorgesehen sein. Das Vehikel 28 für die Newcastle-Disease-Viren 32 (vgl. gestrichelt gezeichneter oberer Halbmond) und das Vehikel 28' für die anti-idiotypischen Antikörperfragmente 12 (vgl. gestrichelt gezeichneter unterer Halbmond). Alternativ könnten auch nur die anti-idiotypischen Antikörperfragmente 12 oder die Newcastle-Disease-Viren 32 ein Vehikel 28, 28' aufweisen (nicht im Detail gezeigt). Auch bei dieser Figur ist zur besseren Darstellbarkeit lediglich ein Antikörperfragment 12, ein Virus 32 und je ein Vehikel 28, 28' gezeigt.

FIG 15 stellt ein zweites alternatives pharmazeutisches Kombinationspräparat 10'' zu einer Unterdrückung einer humoralen Immunantwort gegen einen therapeutischen Organismus 24, dar. Das Kombinationspräparat 10'' unterscheidet sich von dem Kombinationspräparat 10 der FIG 4 bis 9 dadurch, dass es als eine verabreichbare Einheit 54 zusätzlich zu einer Vielzahl von Organismen 24 bzw. von Mikroorganismen 30, beispielsweise in der Form von Newcastle-Disease-Viren 32, eine Vielzahl von viralen Vektoren 68 mit je einem Polypeptid 38 aufweist, das das anti-idiotypisches Antikörperfragment 12 codiert (zur besseren Darstellbarkeit ist lediglich ein Vektormolekül und ein Virus 32 gezeigt). Zudem weist das Kombinationspräparat 10'' einen pharmazeutisch verträglichen Exzipienten 26 sowohl für die Newcastle-Disease-Viren 32 und die Vektoren 68 auf. Alternativ enthält das Kombinationspräparat 10'' ein pharmazeutisch verträgliches Vehikel 28 für die Newcastle-Disease-Viren 32 (vgl. gestrichelt gezeichneter Halbmond). Ferner könnten zwei Vehikel 28, 28' vorgesehen sein. Hierbei stellt der Vektor 68 das Vehikel 28' für das Polynukleotid 38 dar.

Alternativ könnten auch Vektoren 68 mit dem Polypeptid 38, das das anti-idiotypisches Antikörperfragment 12 codiert, in das Virus 32 selbst integriert sein, wie dies durch einen gestichelten Kreis symbolisiert ist. Auch hier ist zur besseren Darstellbarkeit lediglich ein Vektor 68 gezeigt.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 10: Kombinationspräparat
- 12: anti-idiotypisches Antikörperfragment
- 14: Erkennungsstelle
- 16: Struktur
- 18: Teil
- 20: anti-Epitop Antikörperfragment
- 22: Epitop
- 24: Organismus
- 26: Exzipient
- 28: Vehikel
- 30: Mikroorganismus
- 32: Virus
- 34: Wirtszelle
- 36: Neuraminidase
- 38: Polynukleotid
- 40: Körper
- 42: Tumor
- 44: Oberfläche
- 46: Membranprotein
- 48: Haemagglutinin
- 50: Rezeptor
- 52: Zellmembran
- 54: verabreichbare Einheit
- 56: Antikörper(murin)
- 58: Antikörper (chimär)
- 60: Antikörper (humanisiert)
- 62: Kette
- 64: Kette
- 66: Fc-Teil
- 68: Vektor

## Patentansprüche

1. Pharmazeutisches Kombinationspräparat (10) zu einer Unterdrückung einer humoralen Immunantwort gegen einen therapeutischen Organismus (24), enthaltend:
- Zumindest ein anti-idiotypisches Antikörperfragment (12, 12a-12d) mit zumindest einer Erkennungsstelle (14), welche spezifisch zumindest eine Struktur (16) eines variablen Teils (18) eines weiteren Antikörperfragments (20) erkennt, wobei der variable Teil (18) des weiteren Antikörperfragments (20) so ausgeführt ist, dass er zum Erkennen von zumindest einem Epitop (22) zumindest des Organismus (24) vorbereitet ist; und
- mindestens einen pharmazeutisch verträglichen Exzipienten (26) zumindest für das zumindest eine anti-idiotypische Antikörperfragment (12, 12a-12d) und/oder mindestens ein pharmazeutisch verträgliches Vehikel (28) zumindest für das zumindest eine anti-idiotypische Antikörperfragment (12, 12a-12d).

2. Pharmazeutisches Kombinationspräparat nach Anspruch 1,
**gekennzeichnet durch**,
- zumindest den einen Organismus (24), gegen den der variable Teil (18) des weiteren Antikörperfragments (20) gerichtet ist; und
- mindestens einen pharmazeutisch verträglichen Exzipienten (26) zumindest für den zumindest einen Organismus (24) und/oder mindestens ein pharmazeutisch verträgliches Vehikel (28) zumindest für den zumindest einen Organismus (24).

3. Pharmazeutisches Kombinationspräparat nach Anspruch 1 oder 2,
**dadurch gekennzeichnet,**
**dass** der zumindest eine Organismus (24) ein Mikroorganismus (30), insbesondere ein Virus (32) ist, bevorzugt aus der Gruppe der onkolytischen Viren (32), und/oder dass der zumindest eine Organismus (24) ein Virus (32) ist aus der Gruppe bestehend aus einem Adenovirus, einem Herpes-simplex-Virus (oHSV), einem Reovirus, einem Parvovirus (H1), einem Pockenvirus, einem Masernvirus, einem Sendaivirus, einem Newcastle-Disease-Virus (NDV), einem Picornavirus (pig Enterovirus (PEV), ECHO7-Virus), einem Arbovirus, einem Sindbis-Virus, einem Vesicular stomatitis-Virus (VSV), Myxoma-Virus, einem Aujeszky-Virus (pseudorabies-virus, PRV) und einem Seneca-Valley-Virus.

4. Pharmazeutisches Kombinationspräparat nach einem der Ansprüche 1 bis 4,
**dadurch gekennzeichnet,**
**dass** der zumindest eine Organismus (24) ein Mikroorganismus (30), insbesondere ein Bakterium ist, bevorzugt aus der Gruppe der onkolytischen Bakterien und/oder dass der zumindest eine Organismus ein Bakterium ist aus der Gruppe bestehend aus Salmonella typhimurium, Clostridium und E. coli.

5. Pharmazeutisches Kombinationspräparat nach einem der vorhergehenden Ansprüche,
**dadurch gekennzeichnet,**
**dass** das zumindest eine anti-idiotypische Antikörperfragment (12, 12a-12d) ein murines Antikörperfragment (12, 12a-12d) oder ein chimäres Antikörperfragment (12) ist, insbesondere ein hybrides humanes Antikörperfragment (12) oder ein humanisiertes Antikörperfragment (12), und/oder dass das zumindest eine anti-idiotypisches Antikörperfragment (12, 12a-12d) monoklonalen Ursprungs ist.

6. Pharmazeutisches Kombinationspräparat nach einem der vorhergehenden Ansprüche,
**dadurch gekennzeichnet,**
**dass** das zumindest eine anti-idiotypische Antikörperfragment (12a-12d) ein F(ab')₂-Fragment (12a), ein Fab'-Fragment (12b), ein Fab-Fragment (12c) oder ein scFv-Fragment (12d) ist.

7. Pharmazeutisches Kombinationspräparat nach einem der vorhergehenden Ansprüche,
**dadurch gekennzeichnet,**
**dass** das zumindest eine Epitop (22) eine Struktur des zumindest einen Organismus (24) ist, welche dazu ausgelegt ist, an eine Wirtszelle (34), insbesondere eine entartete Wirtszelle (34), anzudocken.

8. Pharmazeutisches Kombinationspräparat nach einem der vorhergehenden Ansprüche,
**dadurch gekennzeichnet,**
**dass** das zumindest eine anti-idiotypische Antikörperfragment (12, 12a-12d) und der mindestens eine pharmazeutisch verträgliche Exzipient (26) zumindest für das zumindest eine anti-idiotypische Antikörperfragment (12, 12a-12d) und/oder das mindestens eine pharmazeutisch verträgliche Vehikel (28) zumindest für das zumindest eine anti-idiotypische Antikörperfragment (12, 12a-12d), sowie der zumindest eine Mikroorganismus (24) und der mindestens eine pharmazeutisch verträgliche Exzipient (26) zumindest für den zumindest einen Organismus (24) und/oder das mindestens eine pharmazeutisch verträgliche Vehikel (28) zumindest für den zumindest einen Organismus (24), jeweils so ausgeführt sind, dass sie räumlich und/oder zeitlich unabhängig voneinander verabreichbar sind.

9. Pharmazeutisches Kombinationspräparat nach einem der vorhergehenden Ansprüche,
**dadurch gekennzeichnet,**
**dass** das zumindest eine anti-idiotypische Antikörperfragment (12, 12a-12d) so ausgeführt ist, dass es zum Reduzieren einer inhibierenden Aktivität des weiteren Antikörperfragments (20) vorbereitet ist, insbesondere, dass das anti-idiotypische Antikörperfragment (12, 12a-12d) so ausgeführt ist, dass es zum Reduzieren der inhibierenden Aktivität eines Antikörperfragments (20) gerichtet gegen zumindest ein Epitop (22), insbesondere Neuraminidase (36), eines onkolytischen Virus (32), insbesondere Newcastle-Disease-Virus (32), vorbereitet ist.

10. Pharmazeutisches Kombinationspräparat nach einem der vorhergehenden Ansprüche,
**dadurch gekennzeichnet,**
**dass** das zumindest eine anti-idiotypische Antikörperfragment (12, 12a-12d) so ausgeführt ist, dass es zum zumindest teilweisen Ersetzen von Zytostatika vorbereitet ist.

11. Pharmazeutisches Kombinationspräparat nach zumindest einem der vorhergehenden Ansprüche,
**dadurch gekennzeichnet,**
**dass** der zumindest eine Organismus (24) so ausgewählt ist, dass er zum Infizieren zumindest einer entarteten Wirtszelle (34) vorbereitet ist, insbesondere, dass der zumindest eine Organismus (24) ein onkolytisches Virus (32) ist, mit welchem die zumindest eine entartete Wirtszelle (34) infizierbar ist.

12. Pharmazeutisches Kombinationspräparat (10') zu einer Unterdrückung einer humoralen Immunantwort gegen einen therapeutischen Organismus (24), enthaltend:
- Zumindest ein anti-idiotypisches Antikörperfragment (12, 12a-12d) mit zumindest einer Erkennungsstelle (14), welche spezifisch zumindest eine Struktur (16) eines variablen Teils (18) eines weiteren Antikörperfragments (20) erkennt, wobei der variable Teil (18) des weiteren Antikörperfragments (20) so ausgeführt ist, dass er zum Erkennen von zumindest einem Epitop (22) zumindest des Organismus(24), insbesondere eines Mikroorganismus (30), vorbereitet ist;
- zumindest den einen Organismus (24), gegen den der variable Teil (18) des weiteren Antikörperfragments (20) gerichtet ist; und
- mindestens einen pharmazeutisch verträglichen Exzipienten (26) und/oder mindestens ein pharmazeutisch verträgliches Vehikel (28).

13. Pharmazeutisches Kombinationspräparat(10'') zu einer Unterdrückung einer humoralen Immunantwort gegen einen therapeutischen Organismus (24), enthaltend:
- Zumindest ein anti-idiotypisches Antikörperfragment (12, 12a-12d) mit zumindest einer Erkennungsstelle (14), welche spezifisch zumindest eine Struktur (16) eines variablen Teils (18) eines weiteren Antikörperfragments (20) erkennt, wobei der variable Teil (18) des weiteren Antikörperfragments (20) so ausgeführt ist, dass er zum Erkennen von zumindest einem Epitop (22), insbesondere Neuraminidase (36), eines onkolytischen Virus (32), insbesondere Newcastle-Disease-Virus (32), vorbereitet ist;
- zumindest den einen onkolytischen Virus (32), insbesondere New-Castle-Disease Virus, gegen den der variable Teil (18) des weiteren Antikörperfragments (20) gerichtet ist; und
- mindestens einen pharmazeutisch verträglichen Exzipienten (26) und/oder mindestens ein pharmazeutisch verträgliches Vehikel (28).

14. Anti-idiotypisches Antikörperfragment (12, 12a-12d) mit zumindest einer Erkennungsstelle (14), welche spezifisch zumindest eine Struktur (16) eines variablen Teils (18) eines weiteren Antikörperfragments (20) erkennt, wobei der variable Teil (18) des weiteren Antikörperfragments (20) so ausgeführt ist, dass er zum Erkennen von zumindest einem Epitop (22) zumindest eines therapeutischen Organismus (24), insbesondere eines onkolytischen Virus (32), bevorzugt Newcastle-Disease-Virus (32), vorbereitet ist, zur Verwendung als Medikament.

15. Polynukleotid (38) codierend das anti-idiotypisches Antikörperfragment (12, 12a-12d) nach Anspruch 14.
